# EUROPEAN PATENT APPLICATION

(11) **EP 4 279 074 A1**
(43) Date of publication of application: **22.11.2023**
(21) Application number: 22174346.1
(22) Date of filing: 19.05.2022
(51) Int. Cl.: A61K 31/5025, A61P 1/00, A61P 9/00, A61P 11/00, A61P 13/12

(54) **AZOLO COMPOUNDS FOR TREATMENT OF FIBROTIC DISEASES**

(71) Applicant: Georg-August-Universität Göttingen Stiftung Öffentlichen Rechts, Universitätsmedizin, 37075 Göttingen (DE); Georg-August-Universität Göttingen, 37073 Göttingen (DE)
(72) Inventor: TIETZE, Lutz Friedjahn, 37077, Göttingen (DE); MOTHUKU, Shyam Sundar, 502108, Telangana (IN); ZEISBERG, Michael, 37073, Göttingen (DE); HASENFUSS, Gerd, 37077, Göttingen (DE); NYAMSUREN, Gunsmaa, 37075 Göttingen (DE); VOSS, Edgar, 34355, Staufenberg (DE)
(74) Representative: Wichmann, Hendrik

(57) **Abstract**

The present invention relates to an azolo compound of Formula I 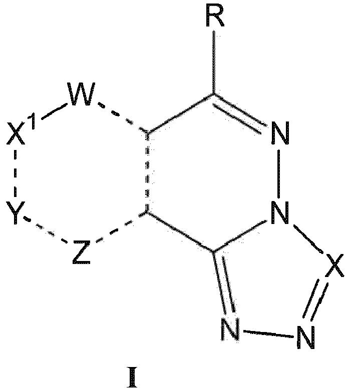 for use in the treatment of fibrosis, wherein the azolo compounds normalizes collagen I mRNA-levels of TGFβ-activated fibroblasts and reverses collagen production to the normal levels that are seen in fibroblasts that are not stimulated.

## Description

The present invention relates to antifibrotic substances, which can be used in the treatment of fibrosis, in particular cardiac fibrosis.

### Technical background

Fibrosis is one of the largest groups of diseases for which - although several potential therapeutic targets have been discussed (Leask, Circulation Research 2010, 106, 1675) - there is still no effective treatment available.

Fibrotic disorders are known to trigger severe diseases if vital organs are involved. Enhanced collagen infiltration in muscles or organs with essential functionality in the body can block their performance completely. Known examples are connective tissue infiltration in liver, kidney, lung, and heart, whereby lung and heart account for the majority of the fibrotic diseases and are responsible for more than 800,000 worldwide deaths annually. Especially myocardial fibrosis resulting in stiffening of heart muscles is one of the primary causes of end stage heart failure (Mozaffarian et al., Heart disease and stroke statistics, Rep. Am. Heart Assoc. 2016, 133, e38-e360). Connective tissue infiltration seen in these fibrotic diseases is characterized by abnormal and overshooting production of collagen by endogenous fibroblast cells.

In spite of high medical need there is no established and curative treatment known. Suggested therapeutic approaches are mostly indirect and depend on the inhibition of common but not specific activation factors of fibroblast collagen production or elimination of hyperactive fibroblasts by toxic intervention points (Hinderer and Schenke-Leyland, Advanced Drug Delivery Reviews 2019, 146, 77; Fan and Guan, Biomaterials Res. 2016, 20, 13/1-13; Frangogiannis, Molecular Aspects of Medicine 2019, 65, 70).

Since elevated blood pressure is known to cause mechanical stress on smooth muscle cells and can lead to fibroblast activation the general use of antihypertensive drugs also may reduce the risk of developing fibrosis. Typical approaches for reducing blood pressure include angiotensin converting enzyme inhibitors, aldosterone antagonists, angiotensin-receptor blockers, and calcium antagonists (R. G. Gourdie, S. Dimmeler, P. Kohl Nat. Rev. Drug Disc. 2016, 15, 620; Y. Shibasaki et al., Hypertens. Res. 2005, 28, 787). Although these drugs can lead to an improvement of the general and especially of the cardiovascular conditions, they do not represent a specific approach to control overshooting fibroblast activity. Their delicate adjustment within complex treatment schemes for hypertension, known drug-drug interactions and the fact that they only relieve one out of many fibroblast activating factors hampers their use as general application for the prevention and treatment of fibrotic diseases. These considerations are also true for finerenone, a nonsteroidal mineralocorticoid receptor antagonist which is supposed to have antifibrotic properties (B. Pitt et al. N Engl J Med 2021,385:2252).

Certain approaches rely on the inhibition of growth factors playing a general role in cell activation and intracellular signaling, mainly targets that have been discussed or aimed at for cancer treatment. These include MMP inhibitors (F.G. Spinale, Phys. Rev. 2007, 87, 1285), inhibitors of mast cell function (S. Oyamada, S. Bianchi, S. Takai, L. M. Chu, J. Pharm. Exp. Ther. 2011, 339, 143), monoclonal antibodies neutralizing MCP1 (S. Hayashidani et al. Circulation 2003, 108, 2134), CXC chemokine ligand 10 (M. Bujak, Circ. Res. 2009, 105, 973), endothelin pathway inhibitors (F. Rodriguez-Pascual, O. Busnadiego, J. Gonzales-Santamaria, Life Sci. 2014, 118, 156), and modulators of WNT-signaling (Daskalopoulos et al. Trends Cardiovasc. Med. 2013, 23, 121). These intervention points do not apply a cell-type specific interaction. Their known side-effects make a success in long-term treatment of fibrosis very unlikely. Compounds that are anti-proliferative do not reflect the important normal function of fibroblasts.

In the healthy heart, fibroblasts are the main cell type involved in the formation and maintenance of connective tissue (K.E. Porter, N.A. Turner Pharmacol. Ther. 2009, 123, 255). This tissue contains cellular and acellular components with the extracellular matrix (ECM) providing a flexible scaffold for individual myocytes and for the heart as a whole (J. B. Caulfield, T. K. Borg, Lab. Invest. 1979, 40, 364). This highly organized collagen rich meshwork, which is laid down by fibroblasts, stabilizes the myocardial wall and supports force transmission, but still allows for intricate patterns of tissue deformations (P.W. Hales et al., Prog. Biophys. Mol. Biol. 2012, 110, 319). After myocardial injury endothelial cells may undergo endothelial-to-mesenchymal transition (EndMT) and thus contribute to fibrosis (E. M. Zeisberg et al., Nat Med. 2007, 13, 952). In this process, endothelial cells lose their endothelial identity and acquire mesenchymal or myofibroblastic markers such as expression of αSMA and secretion of type I collagen. Blocking the transformation of smooth muscle cells and overshooting collagen production after cardiac injury, e.g. after multiple cell death caused by ischemia is an important indication for a non toxic antifibrotic compound.

In 2019, it has been shown that hydralazine and dihydralazine, two drugs being clinically used since 1952 for the treatment of hypertonia, show antifibrotic effects. For instance, dihydralazine in low doses has a protective effect due to an induction of endogenous Tet3/Tdg-mediated DNA-de-methylation activity reversing the aberrant promotor Cpg island methylation. (B. Tampe, D. Tampe, E. M. Zeisberg, G. A. Müller, W. Bechtel-Walz, M. Koziolek, R. Kalluri, M. Zeisberg, EBioMedicine 2015, 2, 19; G. Hasenfuss et al. DE 102019126517 A1 2021.04 01). However, besides their strong antihypertensive action both compounds suffer from high interpatient variance (J.R. Batchelor et al., Lancet 1980, 1(8178), 1107; A. M. Shepherd et al., Clin. Res. 1980, 28, 244A), and drug-induced lupus (M. Bourdi et al., Mol. Pharmacol. 1992, 42, 280; C. Chang, M. Gershwin, J. Autoimmunity 2010, 34, J266).

As a conclusion, so far compounds for the treatment of fibrosis itself are not available, and suggested therapeutic approaches are mostly indirect and depend on the inhibition of common but not specific activation factors of fibroblast collagen production or elimination of hyperactive fibroblasts by toxic intervention points. WO 2016/085981 A1 suggests the use of isoprenoid compounds, in particular geranylgeranylacetone, for inhibiting, reducing, or treating fibrosis. WO 2016/046130 A1 suggests the use of amonoacylglycerol lipase (MGL) inhibitor. WO 2021/089828 A1 discloses nucleic acid ligase inhibitors as possible treatment for fibrosis. This mode of action is generally antiproliferative and as typical cancer treatment suffering from the known unwanted side effects.

A main factor for activating fibroblasts is elevated endogenous TGFβ which induces enhancing of mRNA-levels for collagen I and causes increased collagen production (M. Lodyga and B. Hinz, Semin. Cell. Dev. Biol. 2020, 101, 123; J. M. Carthy, J. Cell. Physiol. 2018, 233(1), 98). This has been proven in cell culture as well as *in vivo* (Lijnen, Pedrov, and Fagard, Molecular Genetics and Metabol. 2000, 71, 418) and therefore the TGFB-stimulation of human renal fibroblast cell lines (tFKIF and tNKF) creates *in vitro* models that represent the pathophysiologic mechanisms of renal interstitial fibrosis and thereby allow the identification of promising treatment approaches (G. A. Müller et al., Exp. Nephrol. 1995, 3, 127).

In view of the above, there exists a need for compounds for use in the treatment of fibrosis, which effectively reduce the formation of fibrotic fibers without detrimental side effects to the patient.

### Summary of the invention

Surprisingly the present inventors found that compounds of the general Formula **I** 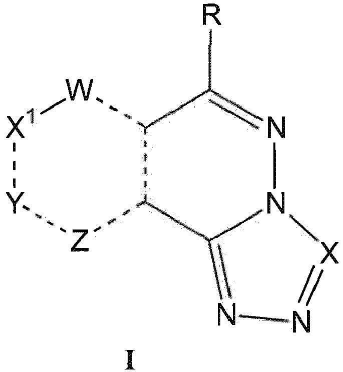 normalize collagen I mRNA-levels of TGFB-activated fibroblasts and reverse collagen production to the normal levels that are seen in fibroblasts, which are not stimulated. Examples **60** to **62** show the biological properties of the azolo compounds of the present invention.

As already mentioned, fibrotic diseases are characterized by abnormal and overshooting production of collagen by endogenous fibroblast cells. Adequate treatment of this disorder should normalize fibroblast activity without toxic effects on cell phenotype and hampering their normal function for regeneration of connective tissue, e.g. after wounding or tissue damage. Unexpectedly we found that compounds of general Formula **I** fulfill this demand. In particular, these structures attenuate elevated collagen production back to normal and reverse the fiber-type phenotype of overactive fibroblasts to the phenotype of non-activated fibroblasts. This is not achieved by a toxic mode of action since it occurs at effective levels without signs of cell toxicity. In this manner compounds of general Formula (**I**) reverse the typical elongated and fiber-type phenotype of activated fibroblasts back to normal. This profound and robust effect typically occurs at concentrations where no attenuation of classical house-keeping enzymes and proteins, e.g. GAPDH (Western-Blot) or reductases (MTT assay) is observed.

Therefore compounds of general Formula **I** have high value for the prevention and treatment of fibrotic diseases with life-threatening outcome or severe reduction of life quality. Examples of such diseases that are not exclusive are kidney fibrosis, liver fibrosis, lung fibrosis, and especially cardiac fibrosis.

The present invention is therefore directed to azolo compound (I) 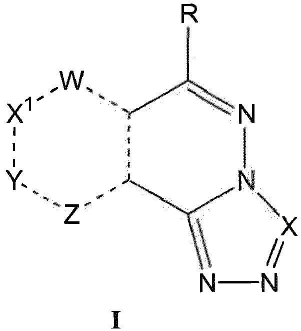 , wherein
- R: is selected from the group consisting of H, D, CN, NHR⁵, NDR⁵, Cl, OR³, SR³ and C₁₋₄-alkyl, in particular methyl;
- X: is N or CR¹, wherein the nitrogen and carbon atom is an aromatic atom;
- W: is selected from the group consisting of a covalent bond, CR⁴, wherein the carbon atom is an aromatic ring atom, CH₂, S, O, N, wherein the nitrogen atom is an aromatic ring atom, NH and NR²;
- X¹: is selected from the group consisting of a covalent bond, CR⁴, wherein the carbon atom is an aromatic ring atom, CH₂, S, O, N, wherein the nitrogen atom is an aromatic ring atom, NH and NR²;
- Y: is selected from the group consisting of a covalent bond, CR⁴, wherein the carbon atom is an aromatic ring atom, CH₂, S, O, N, wherein the nitrogen atom is an aromatic ring atom, NH and NR²;
- Z: is selected from the group consisting of a covalent bond, CR⁴, wherein the carbon atom is an aromatic ring atom, CH₂, S, O, N, wherein the nitrogen atom is an aromatic ring atom, NH and NR²;
wherein 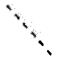 denotes a single bond or a double bond; wherein
W, X¹, Y and Z are members of the same ring and are selected such that the ring consists of five or six members;
W, X¹, Y and Z are selected such that the ring does not comprise more than two nitrogen atoms in the ring, and W, X¹, Y and Z are selected such that the ring does not comprise an acetal, thioacetal, aminal or hydrazine group;
wherein the compound does not comprise a peroxide or disulfide group; and wherein
- R¹: is selected from the group consisting of H, D, CD₃, CHD₂, CH₂D, CF₃, CHF₂, CH₂F, CDF₂, CD₂F, C₁₋₄-alkyl, optionally substituted with OH, halogen, OR³;
- R²: is selected from the group consisting of CHO, COCH₃, COC₂H₅, COC₃H₇, COCH(CH₃)₂, COC₄H₉, COC(CH₃)₃;
- R³: is selected from the group consisting of C₁₋₄-alkyl;
- R⁴: is selected from the group consisting of H, D, F, Cl, methyl, CH₂F, CHF₂, CF₃, OCH₃, OCD₃; and
- R⁵: is C₁₋₄-alkyl, optionally substituted with OH,
for use in a method of treatment or prevention of fibrosis.

The present invention is further directed to pharmaceutical compositions comprising an azolo compound or a mixture of azolo compounds each having the structure (I) 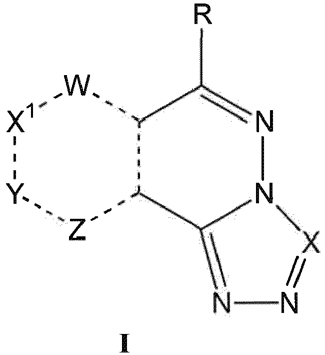 as defined in claim 1 and a pharmaceutically acceptable carrier, preferably for use in the treatment of fibrosis.

### Detailed description of the invention

The present invention relates to new azolo compounds of structure (I) as such, in particular to their use in the treatment of fibrosis:

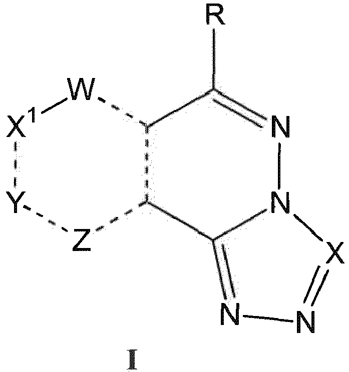

In general, compounds of Formula **I** can be prepared by methods well known in the art (Druey and Rinigier, Helv. Chim. Acta, 34 (1951), 195; Zimmer, Kokosa, and Shah, J. Org. Chem. 40 (1975), 2901). If **X** represents nitrogen (tetrazolo compounds), two typical approaches are shown in scheme I. Thus, a chloropyridazine of Formula **II** is reacted with a metal azide in a dipolar aprotic solvent at higher temperature (method A). Preferred metal azide is sodium azide and favorable conditions are DMF as solvent and temperatures of about 120°C for several hours. Method A is exemplified by examples **2** and **35.** 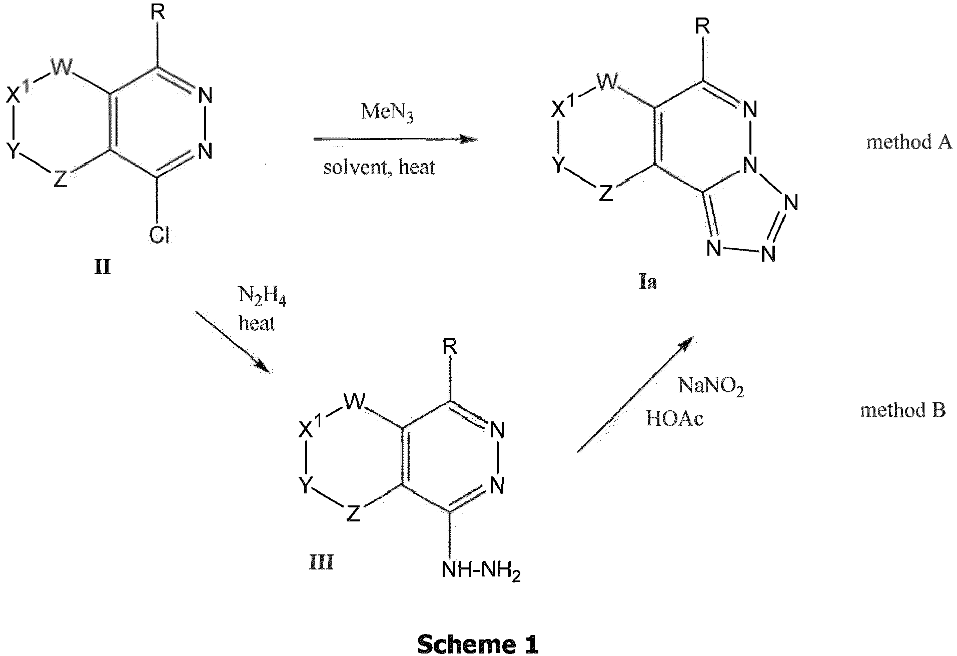 Another approach uses a hydrazine compound of Formula **III** which is reacted with nitrous acid that is formed *in situ* from sodium nitrite under acidic conditions (Badr 1984), (method B). Preferred conditions are diluted acetic acid and slight excess of nitrite at 0°C to room temperature (r.t.). Method B is exemplified by Examples **1** and **17.**

If **X** in Formula **I** represents a substituted carbon atom several known methods can be applied (Druey 1951). Typical approaches are summarized in scheme **2.** Thus compounds of Formula **III** can be reacted with reactive carboxylic acids (method C1) or an activated carboxylic acid derivative (method C2, C3). Reactive carboxylic acids are lower alkyl carboxylic acids (e.g. formic, acetic or propionic acid) or carboxylic acids containing an electron with-drawing substituent at the alpha position e.g. halogen or oxygen). Preferred conditions are using the carboxylic acid as solvent and heating to reflux for several hours. Method C1 is exemplified by examples **10** and **12.**

Activated carboxylic acid derivatives include anhydrides (Badr, El-Sherief, El-Naggar, and Mahgoub, J. Heterocycl. Chem. (1984), 21, 471)(method C2), formic acid esters, ortho esters (method C3) or carboxylic acid derivatives with an appropriate leaving group. Preferred conditions are refluxing with excess of anhydride or reaction with ortho ester at r.t in an alcoholic solvent. Method C2 is exemplified by example **4.** Method C3 is exemplified by Examples **3** and **15.**

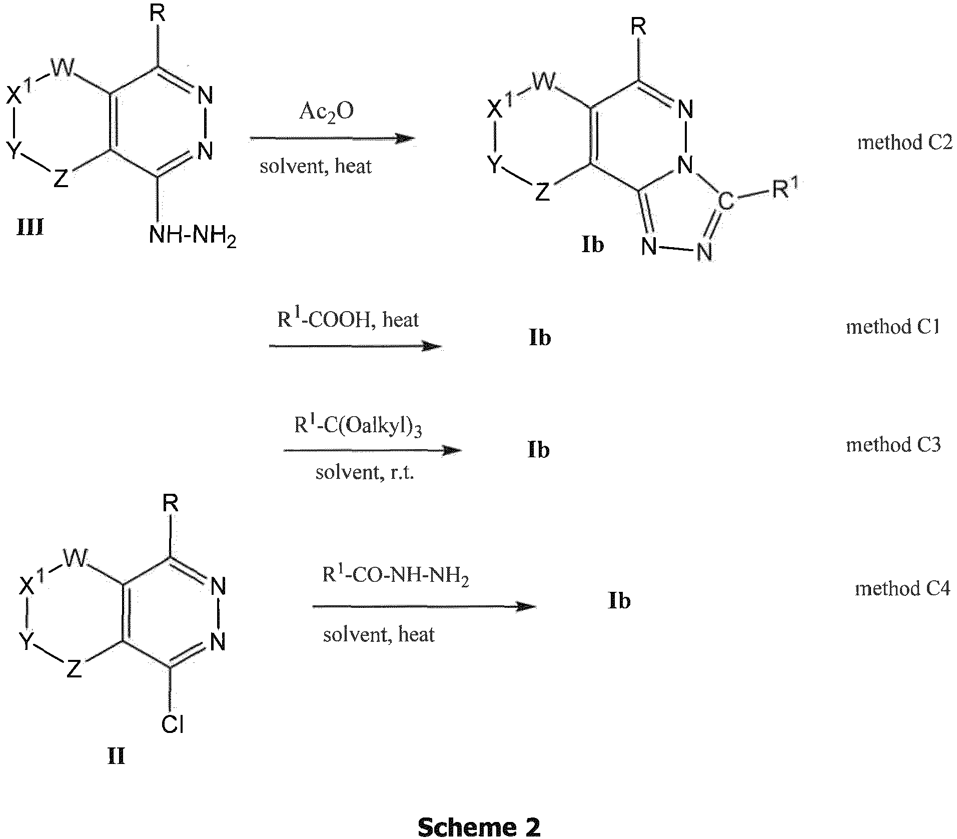

Alternatively, a chloroazine of Formula **II** can be reacted with a carboxylic acid hydrazide at higher temperature in aprotic solvents (method C4). Preferred conditions are at 100-120°C with typical solvents as DMF or dioxane. Method C4 is exemplified by Example **5**, **11**, and **13.**

In a first embodiment, in general Formula (I), R is selected from any one of the group of the group consisting of H, D, CN, NHR⁵, NDR⁵, Cl, OR³, SR³ and C₁₋₄-alkyl. That is, if R = H, H is connected via a covalent bond to the carbon atom of the ring: -H, similarly, when R = D, D is connected via a covalent bond to the carbon atom of the ring: -D, similarly, when R = CN, CN is connected via a covalent bond to the carbon atom of the ring: -CN, similarly, when R = NHR⁵, NHR⁵ is connected via a covalent bond to the carbon atom of the ring: -NHR⁵, similarly for any group of the groups from which R can be selected. Preferably, R is selected from the group consisting of H, CH₃, Cl, CN, OCH₃, OC₂H₅, NHC₂H₄OH, NHCH₃. Further preferably, R is selected from the group consisting of H and CH₃. In the most preferred embodiment R is H.

The term "alkyl" as used herein refers straight chain/linear, or branched hydrocarbon substituents, Examples of alkyl groups include methyl, ethyl, propyl, butyl, isopropyl, sec-butyl, isobutyl, *tert*-butyl,.

In general Formula (I), W can be selected from the group consisting of a covalent bond, CR⁴, wherein the carbon atom is an aromatic ring atom bound (i.e. with a single and a double bond to its neighbor atoms), CH₂, S, O, N, wherein the nitrogen atom is an aromatic ring atom (i.e. bound with a single and a double bond to its neighbor atoms), NH and NR². When W is selected to be a covalent bond (while X¹, Y and Z are not selected to be a covalent bond) , the ring formed by W, X¹, Y, Z and the two bridging carbon atoms is a five membered ring. Preferably, W is selected from the group consisting of CH (bound with a single and a double bond to its neighbor atoms), CH₂ (bound with two single bond to its neighbor atoms) N (bound with a single and a double bond to its neighbor atoms), NH (bound with two single bonds to its neighbor atoms), O, S and a covalent bond.

Similarly, X¹ can be selected from the group consisting of a covalent bond, CR⁴, wherein the carbon atom is an aromatic ring atom (i.e. bound with a single and a double bond to its neighbor atoms), CH₂, S, O, N, wherein the nitrogen atom is an aromatic ring atom (i.e. bound with a single and a double bond to its neighbor atoms), NH and NR². When X¹ is selected to be a covalent bond (while W, Y and Z are not selected to be a covalent bond) , the ring formed by W, X¹, Y, Z and the two bridging carbon atoms is a five membered ring. Preferably, X¹ is selected from the group consisting of CH (bound with a single and a double bond to its neighbor atoms), CH₂ (bound with two double single to its neighbor atoms), N, (bound with a single and a double bond to its neighbor atoms), NH (bound with two single bonds to its neighbor atoms), O, S and a covalent bond.

Similarly, Y can be selected from the group consisting of a covalent bond, CR⁴, wherein the carbon atom is an aromatic ring atom (i.e. bound with a single and a double bond to its neighbor atoms), CH₂, S, O, N, wherein the nitrogen atom is an aromatic ring atom (i.e. bound with a single and a double bond to its neighbor atoms), NH and NR². When Y is selected to be a covalent bond (while W, X¹ and Z are not selected to be a covalent bond) , the ring formed by W, X¹, Y, Z and the two bridging carbon atoms is a five membered ring. Preferably, Y is selected from the group consisting of CH (bound with a single and a double bond to its neighbor atoms), CH₂ (bound with two single bonds to its neighbor atoms) N, (bound with a single and a double bond to its neighbor atoms), NH (bound with two single bonds to its neighbor atoms), O, S and a covalent bond.

Similarly, Z can be selected from the group consisting of a covalent bond, CR⁴, wherein the carbon atom is an aromatic ring atom (i.e. bound with a single and a double bond to its neighbor atoms), CH₂, S, O, N, wherein the nitrogen atom is an aromatic ring atom (i.e. bound with a single and a double bond to its neighbor atoms), NH and NR². When Z is selected to be a covalent bond (while W, X¹ and Y are not selected to be a covalent bond) , the ring formed by W, X¹, Y, Z and the two bridging carbon atoms is a five membered ring. Preferably, Z is selected from the group consisting of CH (bound with a single and a double bond to its neighbor atoms), CH₂ (bound with two single bonds to its neighbor atoms) N, (bound with a single and a double bond to its neighbor atoms), NH (bound with two single bonds to its neighbor atoms), O, S and a covalent bond.

In general Formula (I), 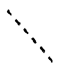 denotes a single bond ( 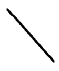 ) or a double bond ( 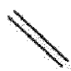 ). While a single bond can be directly adjacent to either a single bond or a double bond, a double bond cannot be selected to be directly adjacent to a neighboring double bond.

As shown in general Formula (I), W, X¹, Y and Z are members of the same ring and are selected such that the ring consists of five or six members. That is, if W is selected to be a covalent bond (single or double), none of X¹, Y or Z can also be selected to be a covalent bond (neither single nor double). Similarly, if X¹ is selected to be a covalent bond, none of W, Y or Z can also be selected to be a covalent bond. Similarly, if Y is selected to be a covalent bond, none of W, X¹ or Z can also be selected to be a covalent bond. Similarly, if Z is selected to be a covalent bond, none of W, Y or X¹ can also be selected to be a covalent bond.

Furthermore, in general Formula (I), W, X¹, Y and Z are selected such that the respective ring formed by W, X¹, Y and Z and the two bridging carbon atoms does not comprise more than two nitrogen atoms in the ring.

In addition, in general Formula (I), W, X¹, Y and Z are selected such that the ring does not comprise an acetal, thioacetal, aminal or hydrazine group and the compound does not comprise a peroxide or disulfide group.

In general Formula (I), R¹ is selected from the group consisting of H, D, CD₃, CHD₂, CH₂D, CF₃, CHF₂, CH₂F, CDF₂, CD₂F, C₁₋₄-alkyl, optionally substituted with OH, halogen, OR³. Preferably, R¹ is selected from the group consisting of H, D, CH₃, CD3, CF₃, CH₂OCH₃, cyclopropyl. That is, if R¹ = H, H is connected via a covalent bond to the carbon atom of the ring: -H, similarly for any group of the groups from which R¹ can be selected.

In general Formula (I), R² is selected from the group consisting of CHO, COCH₃, COC₂H₅, COC₃H₇, COCH(CH₃)₂, COC₄H₉, COC(CH₃)₃. Preferably, R² is selected from the group consisting of CHO and COCH₃. That is, if R² = CHO, CHO is connected via a covalent bond to the nitrogen atom of the ring: -CHO, similarly for any group of the groups from which R² can be selected.

In general Formula (I), R³ is selected from the group consisting of C₁₋₄-alkyl, preferably, C₁₋₄-alkyl, even more preferred methyl. That is, if R³ = C₁ alkyl (i.e. CH₃/methyl), CH₃ is connected via a covalent bond to the sulfur atom: -CH₃, similarly for any group of the groups from which R³ can be selected.

In general Formula (I), R⁴ is selected from the group consisting of H, D, F, Cl, methyl, CH₂F, CHF₂, CF₃, OCH₃, OCD₃. Preferably, R⁴ is selected from the group consisting H, F, Cl and OCH₃, optionally H, F and Cl. That is, if R⁴ = H, H is connected via a covalent bond to the carbon atom of the ring: -CH₃, similarly for any group of the groups from which R⁴ can be selected.

In general Formula (I), R⁵ is C₁₋₄-alkyl, optionally substituted with OH. That is, if R⁵ = C₁ alkyl (i.e. CH₃/methyl), CH₃ is connected via a covalent bond to the nitrogen atom: -CH₃, similarly for any group of the groups from which R⁵ can be selected.

In the context of the present invention, all "embodiments" described herein can be combined with each other. That is, by way of example, if one "embodiment" defines R¹, it can be combined with a further "embodiment" which defines R² or any other substituent.

The azolo compound shown by general Formula (I) is used for the treatment of fibrosis. Examples of fibrosis include but are not limited to kidney fibrosis, liver fibrosis, lung fibrosis and cardiac fibrosis, optionally cardiac fibrosis. Cardiac fibrosis may contribute to heart failure and other cardiac complications in patients with hypertensive heart disease.

The azolo compound shown by general Formula (I) can be used for the treatment of fibrosis by using a single compound or by using mixtures of any two or more thereof. Optionally, the azolo compound is administered in combination with excipients selected from inorganic or organic excipients or combinations thereof. Inorganic excipients include calcium phosphates, calcium carbonate, calcium sulfate, halides, metallic oxides, talc. Organic excipients include carbohydrates such as sugars, sugar alcohols, starch, cellulose such as cellulose ethers, cellulose esters, carboxymethylcellulose, microcrystalline cellulose, petrochemicals, povidones, acrylic polymers.

As discussed above, in general Formula (I), 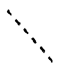 denotes a single bond ( 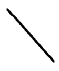 ) or a double bond ( 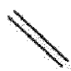 ) or an aromatic ring system. 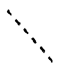 also denotes a single bond ( 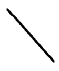 ) or a double bond ( 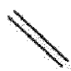 ) with respect to the carbon atoms, which connect the ring comprising W, X¹, Y and Z and the directly connected, neighboring ring. Structures formed by general Formula (I) encompass thus: 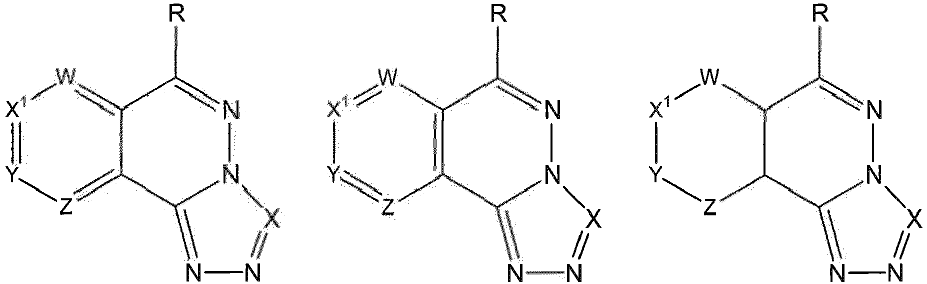 and:

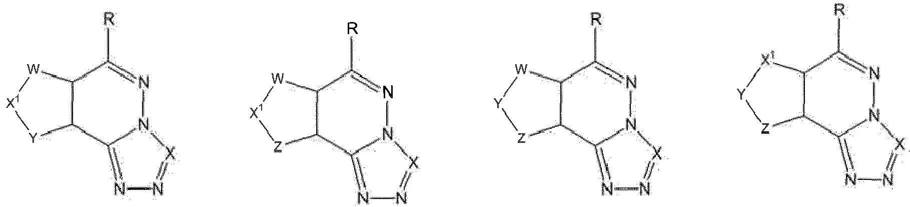

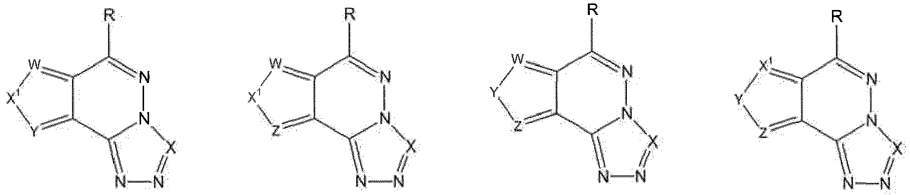

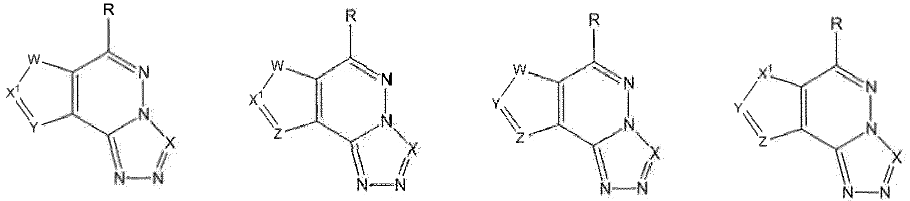

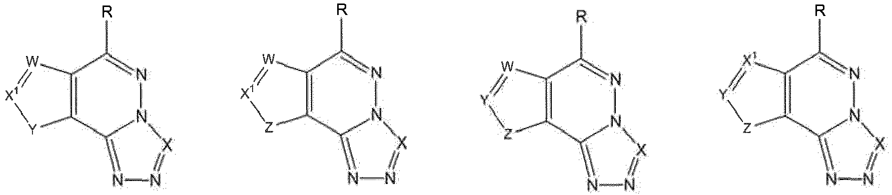

The structures preferably formed by selecting W, X¹, Y and Z can be shown by general Formulae (II a-c) and (III a-d), wherein general Formulae (II a-c) show six-membered rings formed by W, X¹, Y and Z and the two bridging carbon atoms and wherein general Formulae (III a-d) show five-membered rings formed by W, X¹, Y and Z and the two bridging carbon atoms:

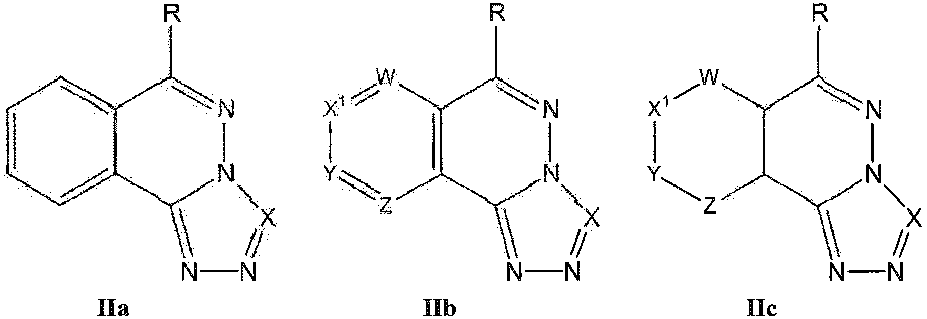

With respect to general Formula (IIb), one of W, X¹, Y and Z is N and the remaining members are CH groups.

With respect to general Formula (IIc), one of W, X¹, Y and Z is NH and the remaining members are CH₂ groups.

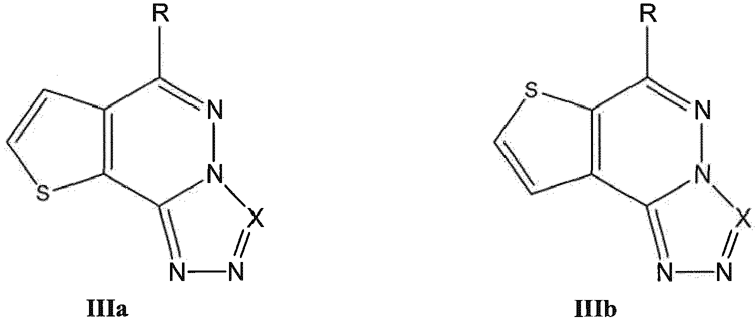

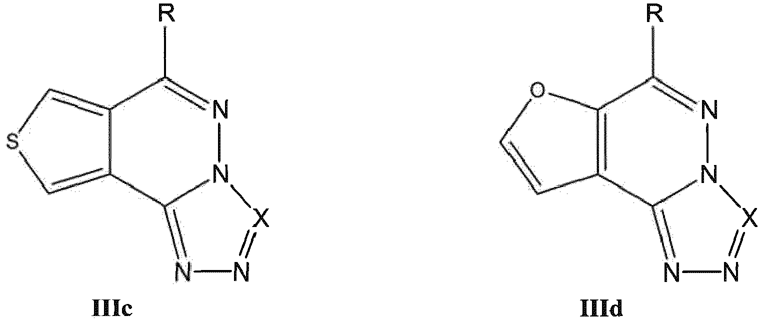

With respect to general Formula (IIIa), Z is S, one of W, X¹ and Y is a covalent bond and the remaining members are CH groups.

With respect to general Formula (IIIb), W is S, one of X¹, Y and Z is a covalent bond and the remaining members are CH groups.

With respect to general Formula (IIIc), either Y or X¹ is S, one of X¹, Y and Z is a covalent bond and the remaining members are CH groups.

With respect to general Formula (IIId), W is O, one of X¹, Y and Z is a covalent bond and the remaining members are CH groups.

It is noted that hydrogen atoms are not shown in Formula (I). Furthermore, any atom in the general Formula (I) can be substituted by any of its isotopes. That is, any hydrogen atom in general Formula (I) (not shown in Formula (I)) can be substituted (partially or fully) by deuterium D. Preferred positions for the substitution of hydrogen with deuterium are as defined in the claims. For example, R can be selected from the group comprising hydrogen and deuterium as defined in the claims. Furthermore, R¹ can be selected from the group comprising hydrogen and deuterium as defined in the claims. Furthermore, any alkyl group of R¹ can optionally be fully or partially be substituted by deuterium instead of hydrogen. Furthermore, R⁴ can be selected from the group comprising hydrogen and deuterium as defined in the claims. Optionally, R¹ = CD₃ or R = D and/or R⁴ = D.

Furthermore, any carbon atom in the general Formula (I) can be substituted (partially or fully) by any of its isotopes, preferably ¹³C. Furthermore, any nitrogen atom in the general Formula (I) can be substituted (partially or fully) by ¹⁵N.

In a second embodiment, R is selected from the group consisting of H, CN, NHR⁵, Cl, OR³, methyl, ethyl, propyl, isopropyl, butyl, isobutyl, *sec*-butyl, *tert*-butyl, cyclopropyl and SR³.

In a further embodiment, R¹ is selected from the group consisting of H, D, CD₃, CHD₂, CH₂D, CF₃, CHF₂, CH₂F, methyl, ethyl, propyl, isopropyl, butyl, isobutyl, *sec-*butyl, *tert*-butyl and cyclopropyl, optionally substituted by OH, Halogen or OR³, optionally, wherein R¹ is selected from the group consisting of H, methyl, CD₃, CF₃, CH₂OH, ethyl, CH₂OCH₃ and cyclopropyl.

In a further embodiment, R³ is selected from the group consisting of methyl, ethyl, propyl, isopropyl, butyl, isobutyl, *sec*-butyl and *tert*-butyl.

In a further embodiment, R⁵ is selected from the group consisting of methyl, ethyl, propyl, isopropyl, butyl, isobutyl, *sec*-butyl, *tert*-butyl, optionally substituted by OH.

In a further embodiment, W, X¹, Y and Z are selected such that the compound does not include a peroxide group and/or such that the compound does not include a disulfide group.

In a further embodiment, X is selected from the group consisting of N and CR¹ and wherein R¹ is selected from the group consisting of H, methyl, CH₂F, CD₂F, CHF₂, CDF₂, and CF₃, preferably, wherein X is CR¹ and R is H or D.

In a further embodiment, W, X¹, Y and Z are selected such that the ring consists of carbon atoms or such that the ring consists of carbon atoms and one heteroatom.

In a further embodiment, W, X¹, Y and Z are selected such that the ring is aromatic or non-aromatic.

In a further embodiment, W, X¹, Y and Z are selected to form a moiety (W···X¹···Y···Z) according to one of the following ring atom sequence: C···C···C···C, N···C···C···C, C···N···C···C, C···C···N···C, C···C···C···N, S···C···C···C, C···S···C···C, C···C···S···C, C···C···C···S, O···C···C···C, C···O···C···C, C···C···O···C, C···C···C···O, C···C···C, N···C···C, C···N···C, C···C···N, S···C···C, C···S···C, C···C···S,, O···C···C, C···O···C, C···C···O, wherein "···" denotes a single bond or a double bond.

In a further embodiment, W, X¹, Y and Z are independently selected such that the ring forms a pyrrolidine, dihydrofuran, dihydrothiophene, pyrrole, furan, thiophene, oxazole, thiazole, tetrahydrobenzene, oxane, thiane, benzene, pyridine, pyran, thiopyran, preferably benzene, pyridine, dihydropyridine thiophene or furan structure.

In a further embodiment, W, X¹, Y and Z are independently selected to form a moiety (W···X¹···Y···Z) selected from the group consisting of CH=CH-CH=CH (to form an aromatic ring), CH=N-CH=CH (to form an aromatic ring), N=CH-CH=CH (to form an aromatic ring), CH=CH-CH=N (to form an aromatic ring), NH-CH₂-CH₂-CH₂, CH₂-NH-CH₂=CH₂, CH₂-NCOCH₃-CH₂-CH₂, S-CH=CH, CH=CH-S, CH-S-CH, CH=CH-O, preferably CH=CH-CH=CH (to form an aromatic ring) or CH-S-CH.

In a further embodiment R = H, X = CH and W, X¹, Y and Z are independently selected to contain one or two of C-F or C-Cl.

In a further embodiment R = H, X = CH and W, X¹, Y and Z are independently selected to form the moiety CH=CH-CH=CH.

In a further embodiment, R=H, X= C-CH₃ or N and W, X¹, Y and Z are independently selected to form the moiety CH=CH-CH=CH.

In a further embodiment, R=H, X= C-CH₃ or N and W, X¹, Y and Z are independently selected to form the moiety CH-S-CH.

In a further embodiment, X and R are independently selected such that the following compound is excluded: X = C-CH₃, R = C-H, and W, X¹, Y and Z = CH.

In a further embodiment, W, X¹, Y and Z are selected to form the moiety CH=CH-CH=CH and R = H and X is N.

In a further embodiment, W, X¹, Y and Z are selected to form the moiety CH=CH-CH=CH and R = H and X is C-CH₃.

In a further embodiment, W, X¹, Y and Z are selected to form the moiety CH=CH-CH=CH and R = H and X is C-H.

In a further embodiment, W, X¹, Y and Z are selected to form the moiety CH-S-CH and R = H and X is C-CH₃.

In a further embodiment, W, X¹, Y and Z are selected to form the moiety CH-S-CH and R = H and X is N.

In a further embodiment, X is C-CH₃, R is H, and W, X¹, Y and Z are forming the group CR⁴=CR⁴-CR⁴=CR⁴ wherein one R⁴ group is selected from the group consisting of F, Cl and OCH₃ and wherein the remaining three R⁴ groups are hydrogens.

In a further embodiment, X is C-CH₃, R is H, and W, X¹, Y and Z are forming the group CR⁴=CR⁴-CR⁴=CR⁴ wherein two R⁴ groups are independently selected from the group consisting of F, Cl and OCH₃ and wherein the remaining two R⁴ groups are hydrogens.

In a further embodiment, the azolo compound (I) is selected from the group consisting of:

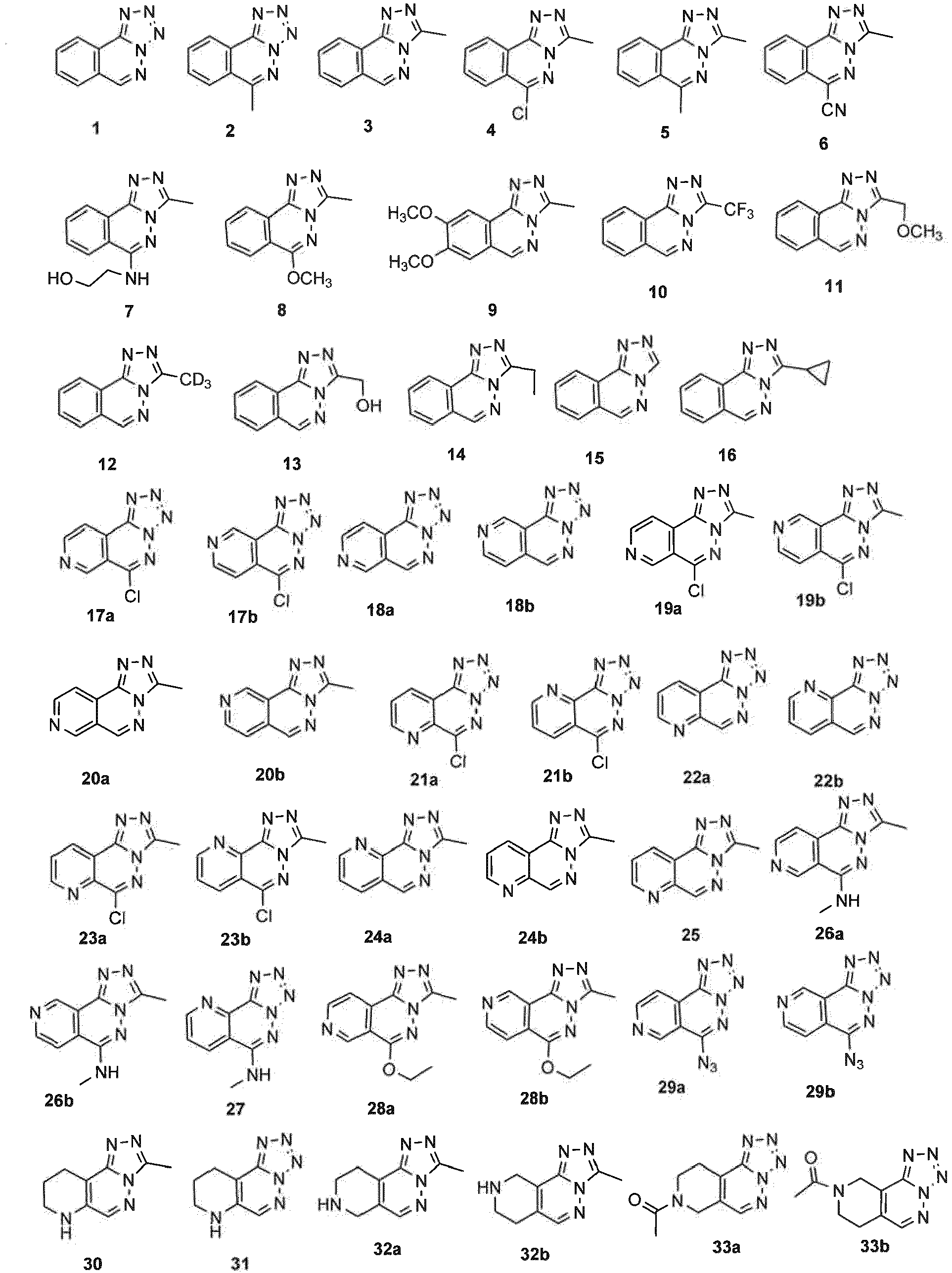

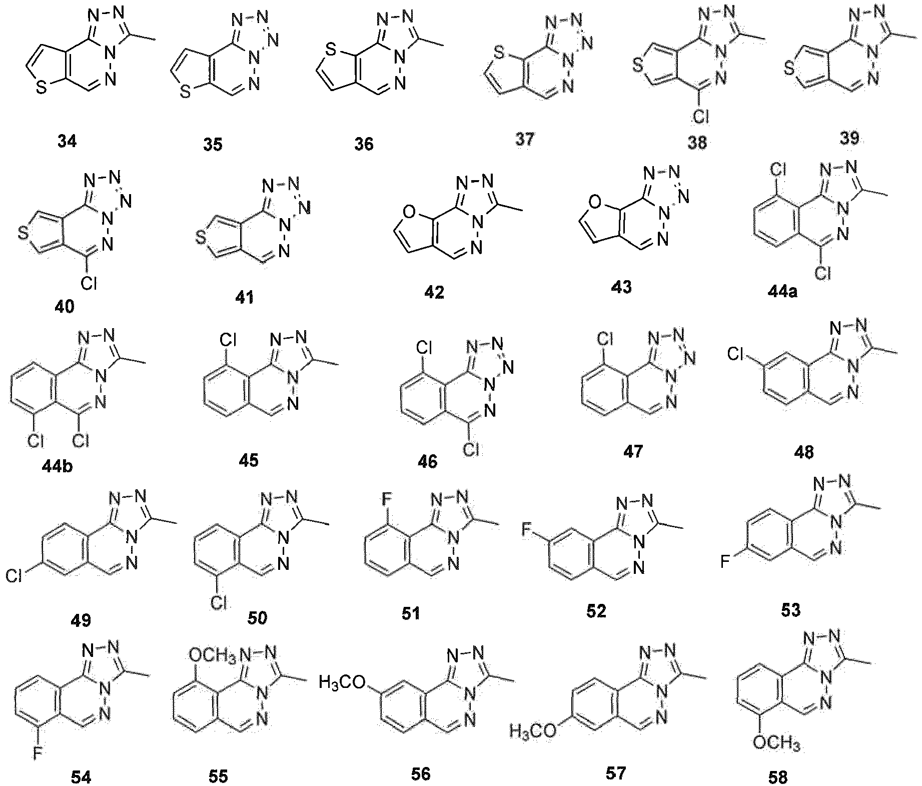

The azolo compound shown by general Formula (I) is used in a method of treatment or prevention of fibrosis. In a further embodiment, fibrosis is selected from the group consisting of kidney fibrosis, liver fibrosis, lung fibrosis and cardiac fibrosis, optionally cardiac fibrosis.

In a further embodiment, one or more of the carbon (C) atoms and nitrogen (N) atoms in the embodiments disclosed herein can be ¹³C isotopes, and N atoms can be ¹⁵N isotopes, wherein it is possible to have compounds with ¹³C isotopes and ¹⁵N isotopes. Furthermore, changing the isotopic composition of H/D, C and N can be applied in the assessment of drug pharmacology to determine the pharmacokinetic profile or mode of action of a drug substance. Secondly, stable isotopes may be used as internal standards for the assessment of drug concentration or its metabolites in blood or urine by analytical quantification LC-MS, HPLC-MS, LC-MS-MS or HPLC-MS-MS methods.

In a further embodiment, provided is a pharmaceutical composition comprising an azolo compound or a mixture of azolo compounds each having the structure (I) 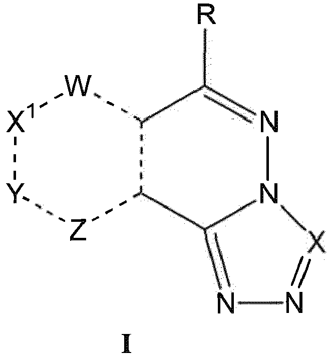 as defined in claim 1 and a pharmaceutically acceptable carrier for use in the treatment of fibrosis. In general Formula (I), " 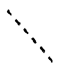 " denotes a single bond ( 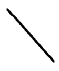 ) or a double bond ( 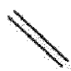 ) or an aromatic ring system. " 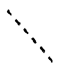 " also denotes a single bond ( 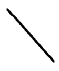 ) or a double bond ( 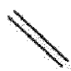 ) with respect to the carbon atoms, which connect the ring comprising W, X¹, Y and Z and the directly connected, neighboring ring.

In a further embodiment, the pharmaceutical composition is an oral solid dosage form and/or the azolo compound is present in an effective amount. Effective amounts of the azolo compound range from 5 to 500 mg, preferably 10 to 200 mg. The compounds according to this invention and their pharmaceutically acceptable salts can be used as medicaments, e.g. in the form of pharmaceutical compositions. The pharmaceutical compositions can be administered orally, e.g. in the form of tablets, coated tablets, dragees, hard and soft gelatine capsules, solutions, emulsions or suspensions. The administration can, however, parenterally, e.g. in the form of injection solutions.

The above-mentioned pharmaceutical compositions can be obtained by processing the compounds according to this invention with pharmaceutically acceptable, inorganic or organic carriers. Lactose, corn starch or derivatives thereof, talc, stearic acids or its salts and the like can be used, for example, as such carriers for tablets, coated tablets, dragees and hard gelatine capsules. Suitable carriers for soft gelatine capsules are, for example, vegetable oils, waxes, fats, semi-solid and liquid polyols and the like. Depending on the nature of the active substance no carriers are, however, usually required in the case of soft gelatine capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical compositions can, moreover, contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

A pharmaceutical composition can comprise e.g. the following:
a) Tablet Formulation (Wet Granulation):

| **Item** | **Ingredients** | **mg/tablet** | | | |
|---|---|---|---|---|---|
| 1. | Compound of formula (I) | 5 | 25 | 100 | 500 |
| 2. | Lactose Anhydrous DTG | 125 | 105 | 30 | 150 |
| 3. | Sta-Rx 1500 | 6 | 6 | 6 | 30 |
| 4. | Microcrystalline Cellulose | 30 | 30 | 30 | 150 |
| 5. | Magnesium Stearate | 1 | 1 | 1 | 1 |
| | Total | 167 | 167 | 167 | 831 |

b) Capsule Formulation:

| **Item** | **Ingredients** | **mg/capsule** | | | |
|---|---|---|---|---|---|
| 1. | Compound of formula (I) | 5 | 25 | 100 | 500 |
| 2. | Hydrous Lactose | 159 | 123 | 148 | - |
| 3. | Corn Starch | 25 | 35 | 40 | 70 |
| 4. | Talc | 10 | 15 | 10 | 25 |
| 5. | Magnesium Stearate | 1 | 2 | 2 | 5 |
| | Total | 200 | 200 | 300 | 600 |

As used herein, the term "a therapeutically effective amount" of a compound means an amount of compound that is effective to prevent, alleviate or ameliorate symptoms of disease or prolong the survival of the subject being treated. Determination of a therapeutically effective amount is within the skill in the art.

The therapeutically effective amount or dosage of a compound according to this invention can vary within wide limits and may be determined in a manner known in the art. Such dosage will be adjusted to the individual requirements in each particular case including the specific compound(s) being administered, the route of administration, the condition being treated, as well as the patient being treated. In general, in the case of oral or parenteral administration to adult humans weighing approximately 70 kg, a daily dosage of about 5 mg to about 500 mg, preferably from about 10 mg to about 200 mg, can be considered as a therapeutically effective amount, although the upper limit may be exceeded when indicated. The daily dosage can be administered as a single dose or in divided doses, or for parenteral administration, it may be given as continuous infusion.

As used herein, a "pharmaceutically acceptable carrier" is intended to include any and all material compatible with pharmaceutical administration including solvents, dispersion media, coatings, antibacterial and antifungal agents, isotonic and absorption delaying agents, and other materials and compounds compatible with pharmaceutical administration. Except insofar as any conventional media or agent is incompatible with the active compound, use thereof in the compositions of the invention are contemplated. Supplementary active compounds can also be incorporated into the compositions.

The compounds according to the present invention may exist and be administrated in the form of their pharmaceutically acceptable salts or esters. The term "pharmaceutically acceptable salt" refers to conventional acid-addition salts that retain the biological effectiveness and properties of the compounds of formula I and are formed from suitable non-toxic organic or inorganic acids. Sample acid-addition salts include those derived from inorganic acids such as hydrochloric acid, sulfuric acid, and those derived from organic acids such as p-toluenesulfonic acid, naphthalenesulfonic acid, naphthalenedisulfonic acid, methanesulfonic acid, ethanesulfonic acid and the like. The chemical modification of a pharmaceutical compound (i.e. a drug) into a salt is a technique well known to pharmaceutical chemists to obtain improved physical and chemical stability, hygroscopicity, flowability and solubility of compounds (cf, e.g. Bastin, R. J., et al., Organic Proc. Res. Dev. 4 (2000) 427-435). Preferred are the pharmaceutically acceptable salts, which are formed with p-toluenesulfonic acid, naphthalenesulfonic acid, naphthalenedisulfonic acid, methanesulfonic acid, sulfuric acid, and hydrochloric acid.

In a preferred embodiment, the azolo compound as defined in the claims is administered in the form of a tablet.

### Examples

### Synthesis of compounds

### Example 1

### Tetrazolo[5,1-a]phthalazine 1

370 mg (5.35 mmol) sodium nitrite dissolved in a minimum amount of water was added at 0°C under stirring to a solution of 982 mg (5.00 mmol) hydralazine hydrochloride and 450 mg (5.50 mmol) sodium acetate in 10 ml 2N acetic acid. A precipitate is formed immediately. After 30 min. the precipitate is isolated by filtration and washed with little ice-cold water. Drying in vacuo at 40°C gave 784 mg (86%) **1.** Extraction of the combined filtrates with ethyl acetate gave additional 60 mg (6%) of **1.** ¹H-NMR (300MHz, CDCl₃): δ = 8.03 (td, *J* = 7.7, 1.3 Hz, 1H), 8.14 (mc, 2H), 8.76 (d, *J* = 7.9 Hz, 1H), 8.96 (s, 1H, 6H).

### Example 2

### 6-Methyl-tetrazolo[5,1-a]phthalazine 2

A mixture of 200 mg (3.1 mmol) sodium azide, 264 mg (1.48 mmol) 1-chloro-4-methylphthalazine, and 10 ml DMF was heated to 120°C for 5 h. After addition of 20 ml water at 0°C a formed pale-yellow precipitate was sucked off. Washing twice with 5 ml ice-cold water and drying in vacuo gave 230 mg (84%) **2** as colorless felty needles. ¹H-NMR (300MHz, CDCl₃): δ = 3.02 (s, 3H, CH₃) 8.03 (td, *J* = 7.5, 1.4 Hz, 1H), 8.10 (td, *J* = 7.5, 1.4 Hz, 1H), 8.22 (d, *J* = 7.6 Hz, 1H), 8.77 (d, *J* = 7.8 Hz, 1H).

### Example 3

### 3-Methyl-1,2,4-triazolo[3,4-a]phthalazine 3

983 mg (5.00 mmol) hydralazine hydrochloride were added to a solution of 1.01 ml (5.50 mmol) triethyl orthoacetate in 10 ml methanol and stirred at r.t. After 15 min. the suspension cleared and a new precipitation started. After 1h additional 10 ml of methanol were added, stirring continued for 1h and the mixture then evaporated. The residue was taken up in 40 ml water and pH adjusted to 7 with solid NaHCO₃. The formed precipitate was isolated by filtration, washed with ice-cold water, and sucked dry. Drying at 50°C in vacuo gave 833 mg (90%) of **3.** Combined filtrates were extracted with 40 ml ethyl acetate to yield additional 30 mg (3%) of **3.** ¹H-NMR (300MHz,): δ = 2.83 (s, 3H, CH₃), 7.79 (ddd, *J* = 8.3, 7.4, 1.2 Hz, 1H), 7.93 (td, *J* = 8.7, 1.2 Hz, 1H), 7.99 (td, *J* = 7.6, 1.2 Hz, 1H), 8.61 (s, 1H), 8.63 (dd, *J* = 9.1, 1.2 Hz, 1H).

### Example 4

### 6-Chloro-3-methyl-1,2,4-triazolo[3,4-a]phthalazine 4

A mixture of 2.58 g (13.0 mmol) 1,4-dichlorophthalazine, 50 ml ethanol and 5.0 ml of 55% hydrazine were heated to reflux for 30 min. After cooling to room temperature (r.t.) a felty precipitate was sucked off and washed twice with ice-cold ethanol. After washing with 10 ml t-butyl methyl ether and 10 ml pentane, the residue was dried in vacuo. Yield 2.23 g (84%) of 1-chloro-4-hydrazinylphthalazine.

An analytical sample (211 mg, 1.08 mmol) of the above was treated with 0.9 ml (1.12 mmol) of 1.25 M HCl in methanol, evaporated and dried in vacuo to give the hydrochloride of 1-chloro-4-hydrazinylphthalazine. ¹H-NMR (300MHz, D₂O): δ= 7.97-8.29 (m, 4H).

Acetic anhydride (0.5 ml, 5 mmol) was added to a suspension of 195 mg (1.00 mmol) 1-chloro-4-hydrazinylphthalazine in 6.0 ml dioxane and the mixture heated for 1h to reflux. After evaporation the residue was treated with 10 ml of ice water and adjusted to pH 7 by addition of sodium bicarbonate. After extraction with 2x 25 ml dichloromethane and evaporation the remaining solid was purified (silica, dichloromethane/methanol 20:1). Yield after drying in vacuum 198 mg **4.** ¹H-NMR (300MHz, CDCl₃): δ = 2.82 (s, 3H, CH₃), 7.86 (ddd, *J* = 8.3, 7.4, 1.2Hz, 1H), 8.00 (td, *J* = 7.6, 1.2 Hz, 1H), 8.26(ddd, *J* = 8.2, 1.3, 0.6 Hz, 1H), 8.67 (ddd, *J* = 8.0, 1.3, 0.7 Hz, 1H).

### Example 5

### 3,6-Dimethyl-1,2,4-triazolo[3,4-a]phthalazine 5

A mixture of 174 mg (0.97 mmol) 1-chloro-3-methylphthalazine, 150 mg acetic acid hydrazide (2.02 mmol) and 5.0 ml dioxane was heated to reflux for 1h. After cooling to r.t. the mixture was evaporated and the residue treated with 30 ml water. Extraction with 2x 30 ml dichloromethane and evaporation gave a residue of 219 mg. After purification by flash chromatography on silica (eluent dichloromethane/methanol 20:1) 176 mg (90%) of the title compound **5** were obtained. ¹H-NMR (300MHz, CDCl₃): δ = 2.80 (s, 3H, CH₃), 2.83 (s, 3H, CH₃), 7.78 (ddd, *J* = 8.6, 7.4, 1.3 Hz, 1H), 7.90 (td, *J=* 7.7, 1.2 Hz, 1H) 7.98 (ddd, *J* = 8.0, 7.7, 1.2 Hz, 1H), 8.64 (ddd, *J* = 8.0, 1.4, 0.6 Hz, 1H).

### Example 6

### 3-Methyl-1,2,4-triazolo[3,4-a]phthalazine-6-carbonitrile 6

A mixture of 135 mg (1.20 mmol) potassium cyanide, 127 mg (0.58 mmol) **4** and 5.0 ml DMSO was heated to 100°c for 2h. After cooling to r.t. 20 ml water was added and the mixture extracted twice with 50 ml dichloromethane. The combined dichloromethane extracts were washed with 10 ml water, dried over sodium sulfate and evaporated. Purification by column chromatography over silica (eluent dichloromethane/methanol 20:1) and drying in vacuo gave 110 mg (83%) **6.** ¹H-NMR (300MHz, CDCl₃): δ = 2.87 (s, 3H, CH₃), 7.96 (ddd, J= 8.3, 7.4, 1.2 Hz, 1H), 8.09 (ddd, *J* = 8.0, 7.7, 1.2 Hz, 1H), 8.27 (dt, *J=* 8.3, 1.0 Hz, 1H), 8.71 (dt, *J* = 8.1, 1.0 Hz, 1H).

### Example 7

### 2-[(3-methyl-1,2,4-triazolo[3,4-a]phthalazin-6-yl)aminol-ethanol 7

A mixture of 70 mg (mmol) **4** (DHY5), 0.6 ml ethanolamine, and 3.0 ml dioxane was kept at 100°C. A precipitate was formed after 20 min. and heating continued for 2h. After evaporation the residue was purified by chromatography on silica (eluent dichloromethane/methanol 20:1→10:1). Yield 62 mg (80%) of **7.** ¹H-NMR (300MHz, d₄-acetic acid): δ = 2.66 (s, 3H, CH₃), 3.73 (t, *J* = 5.2 Hz, 2H), 4.03 (t, *J* = 5.2 Hz, 2H), 7.79 (t, *J* = 7.8 Hz, 1H), 7.88 (t, *J* = 7.5 Hz, 1H), 8.05 (d, *J* = 8.1 Hz, 1H), 8.51 (d, *J* = 7.2 Hz, 1H).

### Example 8

### 6-Methoxy-3-methyl-1,2,4-triazolor3,4-a]phthalazine 8

Compound **4** from example **4** (0.050 g, 0.22 mmol, 1.0 equiv.) was dissolved in MeOH (3 mL), then Pd-C (10%) (50 mg) was added. The reaction mixture was stirred for 24 h at room temperature. The product was collected by filtration, and dried under vacuum (41 mg, 84%) **8**. ¹H-NMR (400 MHz, CDCl₃): δ = 8.56 (ddd, *J* = 8.0, 1.3, 0.7 Hz, 1H), 8.14 (ddd, *J* = 8.1, 1.3, 0.7 Hz, 1H), 7.87 (ddd, *J* = 8.0, 7.3, 1.2 Hz, 1H), 7.72 (ddd, *J* = 8.1, 7.3, 1.3 Hz, 1H), 4.18 (s, 3H), 2.74 (s, 3H). ¹³C-NMR (126 MHz, CDCl₃): δ= 158.4, 147.8, 142.6, 133.7, 130.4, 125.1, 124.7, 123.22, 118.69, 55.30, 9.92. HRMS (ESI): cal. mass C₁₁H₁₀N₄O [M-1]⁻ 213.0782, found [M-1]⁻213.0781.

### Example 9

### 8,9-Dimethoxy-3-methyl-1,2,4-triazolo[3,4-a]phthalazine 9

A mixture of 57 mg (0.25 mmol) 1-chloro-6,7-dimethoxypthalazine, 100 mg (1.3 mmol) acetic acid hydrazide, and 6.0 ml dioxane were heated to reflux for 12 h. After evaporation the residue was purified by column chromatography on silica (dichloromethane/methanol 20:1) to give 38 mg (61%) of **9.** ¹H-NMR (300MHz, CDCl₃): δ = 2.80 (s, 3H, CH₃), 4.04 (s, 3H, OCH₃), 4.10 (s, 3H, OCH₃), 7.22 (s, 1H), 7.96 (s, 1H), 8.48 (s, 1H).

### Example 10

### 3-Trifluoromethyl-1,2,4-Triazolo[3,4-a]phthalazine 10

237 mg (1.21 mmol) hydralazine hydrochloride and 5.0 ml trifluoroacetic acid were heated to 70°C for 4 h. The mixture was evaporated and 5.0 ml water added. After adjustment to pH 5 the product was extracted with 2x 20 ml dichloromethane. Drying (Na₂SO₄) and evaporation gave 273 mg (92%) yellow solid that was purified by column chromatography on silica (dichloromethane/methanol 50:1). Yield 263 mg (89%) colorless **10.** ¹H-NMR (300MHz, CDCl₃): δ = 7.97 (dd, *J* = 8.4, 7.0 Hz, 1H), 8.07 (d, *J* = 8.0 Hz, 1H), 8.09 (td, *J* = 7.2, 1.4 Hz, 1H), 8.79 (dd, *J =* 7.9, 1.2 Hz), 8.84 (s, 1H).

### Example 11

### 3-Methoxymethyl-1,2,4-triazolo[3,4-a]phthalazine 11

100 mg (mmol) 1-Chlorophthalazine and 200 mg (mmol) methoxyacetic acid hydrazide were suspended in 5.0 ml dioxane and heated to reflux for 2 h. The reaction mixture was evaporated and the residue distributed between 20 ml water and 20 ml dichloromethane. The water phase was extracted with 20 ml dichloromethane and the combined organic phases washed with 10 ml water. After drying over sodium sulphate and evaporation, the residue was purified over a silica column (eluent: ethyl acetate). Yield 112 mg (82%) of **11.** ¹H-NMR (300MHz, CDCl₃): δ = 3.49 (s, 3H, OCH₃), 5.05 (s, 2H, CH₂O), 7.82 (td, *J* = 7.5, 1.2 Hz), 7.95 (d, *J* = 7.5 Hz, 1H), 7.96 (td, *J* = 7.5, 1.1Hz, 1H), 8.67 (dd, *J* = 7.1, 1.2 Hz, 1H), 8.68 (s, 1H).

### Example 12

### 3-D₃-Methyl-1,2,4-triazolo[3,4-a]phthalazine 12

314 mg (1.60 mmol) Hydralazine hydrochloride and 2.5 ml acetic acid-d₄ were heated to 120°C for 6 h. After evaporation 5.0 ml water was added and the pH brought to 7 by addition of NaHCO₃. Extraction with 2x 50 ml dichloromethane, drying (Na₂SO₄), and evaporation gave a yellow solid containing d3-acetic acid. Purification double column chromatography on silica (first column dichlormethane/methanol 30:1, second column ethyl acetate yielded 236 mg (75%) **12** as colorless solid. ¹H-NMR (300MHz, CDCl₃): δ = 7.75(t, *J* = 7.4 Hz, 1H), 7.88 (m, 2H), 8.56 (d, *J* = 7.4 Hz, 1H), 8.57 (s, 1H).

### Example 13

### 1,2,4-Triazolo[3,4-a]phthalazin-3-yl-methanol 13

302 mg (1.83 mmol) 1-chlorophthalazine and 349 mg (3.87 mmol) hydroxyacetic acid hydrazide were added to 5.0 ml dioxane and heated to reflux for 4 h. After evaporation the residue was taken up in 10 ml water and the resulting suspension extracted with 7x with 50 ml dichloromethane. After drying (Na₂SO₄) and evaporation the residue was purified by column chromatography over silica (dichloromethane/methanol 20:1). Yield 349 mg (90%) **13** as colorless solid. ¹H-NMR (300MHz, CDCl₃): δ = 4.95 (s, 2H), 5.70 (br, s, 1H, OH), 7.94 (td, *J* = 7.7, 1.2 Hz, 1H), 8,06 (td, *J* = 7.6, 1.2 Hz, 1H), 8.22 (d, *J* = 7.8 Hz, 1H), 8.51 (d, *J* = 7.9 Hz, 1H), 9.09 (s, 1H).

### Example 14

### 3-Ethyl-1,2,4-triazolo[3,4-a]phthalazine 14

A solution of 376mg hydralazine hydrochloride (1.91 mmol) in 3.0 ml propionic acid is heated to 120°C for 4 h. After cooling to r.t. 20 ml water was added and the solution neutralized by addition of NaHCO₃. Extraction with 20 ml dichloromethane, drying over Na₂SO₄ evaporation gave a yellow-brown residue. Purification by column chromatography over silica (dichloromethane → dichloromethane/methanol 20:1 gto give 38 mg of **14.** ¹H-NMR (300MHz, CDCl₃): δ = 1.50(t, *J* = 7.6 Hz, 3H, CH₃), 3.23 (q, *J* = 7.6 Hz, 2H, CH₂), 7.78 (ddd, *J* = 7.2, 6.8, 1.2 Hz, 1H), 7.91 (d, *J* = 7.4 Hz, 1H), 7.92 (t, *J* = 7.9 Hz, 1H), 8.52 (s, 1H), 8.53 (d, *J* = 7.9 Hz, 1H).

### Example 15

### 1,2,4-Triazolo[3,4-a]phthalazine 15

0.55 ml (3.0 mmol) triethyl orthoformate were added to a solution of 500 mg (2.54 mmol) hydralazine hydrochloride in 5.0 ml methanol. Stirring at r.t. was continued for 2 h. A fine crystalline precipitate was formed within 1h. After evaporation 5 ml water was added and pH adjusted to pH 5 with solid NaHCO₃. After extraction with 3x 20 ml dichloromethane the organic phase was dried (Na₂SO₄) and evaporated. Drying in vacuo gave 401mg (87%) of **15.** ¹H-NMR (300MHz, CDCl₃): δ = 7.83 (ddd, *J* = 8.2, 7.3, 1.2 Hz, 1H), 7.96(d, *J* = 7.9 Hz, 1H), 7.98 (td, *J* = 7.5, 1.2 Hz, 1H), 8.64 (s, 1H), 8.68 (dd, *J* = 7.6, 1.2 Hz, 1H), 9.04 (s, 1H).

### Example 16

### 3-Cyclopropyl-[1,2,4]triazolo[3,4-a]phthalazine 16

178 mg NaHCO₃ were added to a solution of 180 mg of cyclopropane carboxylic acid in 20 ml methanol and evaporated. To the residue were added 188 mg hydralazine hydrochloride, 500 mg cyclopropane carboxylic acid, and 10 ml dioxane. The mixture was heated to reflux for 6 h. After twice column chromatography on silica (first: ethyl acetate / hexane 2:1; second dichloromethane / methanol 50:1) 18 mg of **16** were obtained.

¹H NMR (300 MHz, CDCl₃) δ 8.63 (d, *J* = 7.9 Hz, 1H), 8.60 (s, 1H), 7.92 (t, *J* = 7,5 Hz 1H), 7,91 (d, *J* = 7,7 Hz, 1H), 7.79 (dd, *J* = 8.5, 6.6 Hz, 1H), 2.50 (tt, *J* = 8.5, 5.0 Hz, 1H), 1.39 (dt, *J* = 6.4, 3.3 Hz, 2H), 1.21 (dt, *J* = 8.5, 3.3 Hz, 2H).

### Example 17

### 6-Chloropyrido[4,3-d]tetrazolo[1,5-b]pyridazine 17a and 6-chloropyrido[3,4-d]tetrazolo[1,5-b]pyridazine 17b

### 2,3-Dihydropyrido[3,4-d]pyridazin-1,4-dione (i)

3,4-Pyridinedicarboxylic acid (8.61 g, 51.51 mmol, 1.0 equiv.) was dissolved in acetic acid anhydride (30 mL, 319.42 mmol, 6.2 equiv.), and the mixture was heated to reflux with stirring for 1 h. After cooling to room temperature, 30 mL hydrazine (55% in water) was added and the mixture was refluxed with stirring for 4 h. The product was collected by suction filtration, washed with water and dried, to yield an amorphous solid (i) (7.94 g, 95%). ¹H NMR (300 MHz, DMSO): δ= 11.90 (s, 2H), 9.32 (s, 1H), 9.02 (d, *J* = 5.3 Hz, 1H), 7.89 (d, *J* = 5.3 Hz, 1H).

### 1,4-Dichloropyrido[3,4-d]pyridazine (ii)

A mixture of 2,3-dihydropyrido[3,4-d]pyridazin-1,4-dione (i) (6.52 g, 39.96 mmol, 1.0 equiv.) (0.04 mol) and phosphorus oxychloride (18.6 mL, 189.84 mmol, 4.75 equiv.) in pyridine (6.46 mL, 79.93 mmol, 2.0 equiv.) was heated to reflux and stirred for 8 h. The mixture then was poured into a slush of 400 g of ice, then neutralized with NaHCO₃ and then extracted with ethyl acetate (3 × 100 mL). The organic phase was separated, washed with water, dried with Na₂SO₄ and evaporated to dryness. The crude product was purified by column chromatography using dichloromethane to give (ii) as a yellow amorphous solid (4.01 g, 50%). ¹H NMR (300 MHz, CDCl₃), main isomer: δ= 9.75 (d, *J* = 1.0 Hz, 1H), 9.24 (d, *J* = 5.6 Hz, 1H), 8.07 (dd, *J* = 5.6, 1.0 Hz, 1H).

### 4-Chloro-1-hydrazinylpyrido[3,4-d]pyridazine (iiia) and 1-chloro-4-hydrazinylpyrido[3,4-d]pyridazine (iiib)

To a solution of 1,4-dichloropyrido[3,4-d]pyridazine (ii) (1.0 g, 4.99 mmol, 1.0 equiv.) in methanol (15 mL) was added slowly hydrazine (55% in water) (0.32 g, 9.99 mmol, 2.0 equiv. and the reaction mixture was refluxed for 10 min to give a yellow precipitation. The product was collected by suction filtration and dried to give the product as a mixture of isomers (iiia/iiib), which was used subsequently used without further purification. HRMS (ESI): cal. mass C₇H₆N₅Cl [M+1]⁺ 196.0390, found [M+1]⁺ 196.0382.

### 6-Chloropyrido[4,3-d]tetrazolo[1,5-b]pyridazine 17a and 6-chloropyrido[3,4-d]tetrazolo[1,5-b] pyridazine 17b

A solution of the mixture of isomers (iiia and iiib) (0.20 g, 1.02 mmol, 1.0 equiv.) in acetic acid (5 mL, 2N) was cooled with ice-water and then treated dropwise with stirring with a cold aqueous solution of NaNO₂ (84 mg, 1.22 mmol, 1.2 equiv. in 2 mL water). Stirring was continued at room for 1 h. The product was extracted with dichloromethane (3 × 25 mL). Organic phase was dried with Na₂SO₄ and evaporated to give amorphous solid **17a/17b** (181 mg, 86%). ¹H NMR (300 MHz, DMSO): δ= 10.05 (s, 1H), 9.77 (s, 0.4H), 9.36-9.34 (m, 1.4H), 8.65 (d, *J* = 5.4 Hz, 0.4H), 8.36 (d, *J* = 5.4 Hz, 1H). HRMS (ESI): cal. mass C₇H₃N₆Cl [M+1]⁺ 207.0186, found [M+1]⁺ 207.0181.

### Example 18

### Pyrido[4,3-d]tetrazolo[1,5-b]pyridazine 18a and pyrido[3,4-d]tetrazolo[1,5-b]pyridazine 18b

To a solution of the mixture of isomers **17a/17b** (50 mg, 0.24 mmol, 1.0 equiv.) in ethanol (3 mL) was added slowly hydrazine (55% in water) (15.5 mg, 0.48 mmol, 2.0 equiv.) and the reaction mixture was refluxed for 2 h to give a precipitation. The product was collected by suction filtration and dried. Then, a solution of 0.15 M potassium trimethylsilanolate (TMSOK) (31 mg, 0.24 mmol, 1.0 equiv.) in water (1.6 mL) was added and the formed slurry was stirred for 24 h at room temperature. The resulting mixture was extracted with dichloromethane (4x20 mL). Organic phase was dried with Na₂SO₄ and evaporated to give an amorphous solid as a mixture of isomers **18a/18b** (14 mg, 34%). ¹H NMR (500 MHz, CDCl₃) δ= 10.15 (d, *J* = 0.9 Hz, 1H), 9.56 (d, *J* = 1.0 Hz, 0.4H), 9.29 - 9.27 (m, 1.4H), 9.11 (d, *J* = 0.8 Hz, 0.4H), 9.03 (d, *J* = 0.7 Hz, 1H), 8.58 (dt, *J* = 5.4, 0.9 Hz, 0.4H), 7.99 (dt, *J* = 5.4, 0.8 Hz, 1H). ¹³C NMR (126 MHz, CDCl₃): δ= 153.64, 152.38, 151.05, 148.06, 147.99, 147.73, 140.67, 140.24, 128.74, 127.65, 119.80, 119.19, 117.09, 116.36. HRMS (ESI): cal. mass C₇H₅N₆ [M+1]⁺ 173.0570, found [M+1]⁺ 173.0568, cal. mass C₇H₄N₆Na [M+Na]⁺ 195.0390, found [M+Na]⁺ 195.0387.

### Example 19

### 6-Chloro-3-methylpyrido[4,3-d][1,2,4]triazolo[4,3-b]pyridazine 19a and 6-chloro-3-methylpyrido[3,4-d][1,2,4]triazolo[4,3-b]pyridazine 19b

A mixture of isomers (iiia/iiib, from **example 17**) (0.20 g, 1.02 mmol, 1.0 equiv.) and triethyl orthoacetate (0.25 g, 1.53 mmol, 1.5 equiv.) was refluxed in methanol (5 mL) for 20 min to give a solid product. The product was collected by suction filtration and dried to give a mixture of isomers **19a/19b** (0.16 g, 74%). ¹H NMR (300 MHz, DMSO): δ= 9.82 (d, *J* = 0.9 Hz, 1H), 9.52 (d, *J* = 0.9 Hz, 0.6H), 9.16 (dd, *J* = 7.2, 5.4 Hz, 1.7H), 8.41 (dd, *J* = 5.3, 1.0 Hz, 0.6H), 8.14 (dd, *J* = 5.5, 0.9 Hz, 1H), 2.71 (broad signal, 5H). HRMS (ESI): cal. mass C₉H₆ClN₅ [M+1]⁺ 220.0384, found [M+1]⁺ 220.0386, cal. mass C₉H₆ClN₅Na [M+Na]⁺ 242.0204, found [M+Na]⁺ 242.0206.

### Example 20

### 3-methylpyrido[4,3-d][1,2,4]triazolo[4,3-b]pyridazine 20a and 3-methylpyrido[3,4-d][1,2,4]triazolo [4,3-b]pyridazine 20b

To a solution of the mixture of isomers **19** (50 mg, 0.22 mmol, 1.0 equiv.) in ethanol (3 mL) was added slowly N₂H₄.H₂O (55%) (14.5 mg, 0.44 mmol, 2.0 equiv.) and the mixture was refluxed for 2 h to give a precipitation. The product was collected by suction filtration and dried. Then, a solution of 0.15 M TMSOK (30 mg, 0.24 mmol, 1.0 equiv.) in water (1.5 mL) was added and the slurry was stirred for 24 h at room temperature. The resulting mixture was extracted with dichloromethane (4x20 mL). Organic phase was dried with Na₂SO₄ and evaporated to give an amorphous solid **(20a/20b)** (12 mg, 34%). ¹H NMR (300 MHz, CDCl₃): δ= 10.01 (s, 1H), 9.30 (s, 0.5H), 9.07 (d, *J* = 5.3 Hz, 0.5H), 9.08 (d, *J* = 5.3 Hz, 1H), 8.75 (s, 0.5H), 8.67 (s, 1H), 8.45 (d, *J* = 5.3 Hz, 0.5H), 7.75 (d, *J* = 5.3 Hz, 1H), 2.86 (broad signal, 4.5H). ¹³C NMR (126 MHz, CDCl₃): δ= 153.12, 150.74, 150.56, 149.33, 148.85, 146.96, 146.11, 145.77, 140.87, 140.37, 129.13, 127.06, 119.74, 117.87, 117.81, 116.15, 10.04, 9.98. HRMS (ESI): cal. mass C₉H₈N₅ [M+1]⁺ 186.0774, found [M+1]⁺ 186.0771, cal. mass C₉H₇N₅ Na [M+Na]⁺ 208.0594, found [M+Na]⁺ 208.0589.

### Example 21

### 6-Chloropyrido[3,2-d]tetrazolo[1,5-b]pyridazine 21a and 6-chloropyrido[2,3-d]tetrazolo[1,5-b]pyridazine 21b

### 6,7-Dihydropyrido[2,3-d]pyridazine-5,8-dione (iv)

The compound was prepared from diacid according to the example **17**, compound (i). ¹H NMR (300 MHz, DMSO): δ= 11.63 (s, 2H), 9.11 (dd, *J* = 4.5, 1.7 Hz, 1H), 8.46 (dd, *J* = 8.1, 1.7 Hz, 1H), 7.88 (dd, *J* = 8.1, 4.5 Hz, 1H).

### 5,8-Dichloropyrido[2,3-d]pyridazine (v)

The compound was prepared from compound (iv) according to the example **17**, compound (ii). ¹H NMR (300 MHz, CDCl₃): δ= 9.41 (dd, *J* = 4.4, 1.6 Hz, 1H), 8.64 (dd, *J* = 8.4, 1.6 Hz, 1H), 8.02 (dd, *J* = 8.4, 4.4 Hz, 1H). ¹³C NMR (75 MHz, CDCl₃): δ= 154.33, 141.13, 134.38, 129.09, 124.02.

### 6-Chloropyrido[3,2-d]tetrazolo[1,5-b]pyridazine 21a and 6-chloropyrido[2,3-d]tetrazolo[1,5-b] pyridazine 21b

The compound (**21a/21b**) was prepared from compound (v) according to the example **17** compound (iii). ¹H NMR (300 MHz, CDCl₃): δ= 9.42 (dd, *J* = 4.6, 1.6 Hz, 1H), 9.39 (dd, *J* = 4.6, 1.6 Hz, 0.45H), 9.08 (dd, *J* = 8.2, 1.7 Hz, 0.45H), 8.76 (dd, *J* = 8.2, 1.7 Hz, 1H), 8.11 (dd, *J* = 8.2, 4.5 Hz, 0.45H), 8.06 (dd, *J* = 8.2, 4.5 Hz, 1H).

Mixture of isomers were separated by column chromatography.
first eluent isomer: 6-Chloropyrido[3,2-d]tetrazolo[1,5-b]pyridazine **21a.** ¹H NMR (600 MHz, CDCl₃) δ 9.39 (dd, *J* = 4.5, 1.7 Hz, 1H), 9.09 (dd, *J* = 8.1, 1.7 Hz, 1H), 8.09 (dd, *J* = 8.1, 4.5 Hz, 1H). ¹³C NMR (126 MHz, CDCl₃) δ 155.56, 154.35, 141.96, 139.60, 133.25, 129.29, 119.72.
second eluent isomer: 6-chloropyrido[2,3-d]tetrazolo[1,5-b] pyridazine **21b.** ¹H NMR (600 MHz, CDCl₃) δ 9.42 (dd, *J* = 4.5, 1.6 Hz, 1H), 8.76 (dd, *J* = 8.4, 1.6 Hz, 1H), 8.06 (dd, *J* = 8.4, 4.6 Hz, 1H). ¹³C NMR (126 MHz, CDCl₃) δ 157.71, 151.65, 142.40, 140.00, 135.96, 127.80, 121.58.

### Example 22

### Pyrido[3,2-d]tetrazolo[1,5-b]pyridazine 22a (minor isomer) and pyrido[2,3-d]tetrazolo[1,5-b] pyridazine 22b (major isomer)

The compound was prepared from compound **21** according to the example **18.** ¹H-NMR (600 MHz, DMSO): δ= 9.47 (s, 1H), 9.43 (d, *J* = 0.7 Hz, 0.2H), 9.36 (dd, *J* = 4.6, 1.7 Hz, 1H), 9.33 (dd, *J* = 4.5, 1.6 Hz, 0.2H), 9.06 (dd, *J* = 8.2, 1.6 Hz, 0.2H), 8.81 (dd, *J* = 8.1, 1.7 Hz, 1H), 8.16 (dd, *J* = 8.2, 4.6 Hz, 0.2H), 8.11 (dd, *J* = 8.1, 4.6 Hz, 1H). ¹³C-NMR (126 MHz, DMSO): δ= 156.77, 155.57, 151.53, 150.74, 143.20, 142.55, 141.86, 139.83, 137.68, 132.36, 129.15, 127.68, 122.20, 119.41. HRMS (ESI): cal. mass C₇H₅N₆ [M+1]⁺ 173.0570, found [M+1]⁺ 173.0571, cal. mass C₇H₄N₆Na [M+Na]⁺ 195.0390, found [M+Na]⁺ 195.0386.

### Example 23

### 6-chloro-3-methylpyrido[3,2-d][1,2,4]triazolo[4,3-b]pyridazine 23a and 6-chloro-3-methylpyrido[2,3-d][1,2,4]triazolo[4,3-b]pyridazine 23b

The compound (**23a/23b**) was prepared from compound (v) according to the example **19.** ¹H NMR (300 MHz, CDCl₃) δ 9.25 (dd, *J* = 4.6, 1.6 Hz, 0.6H), 9.17 (dd, *J* = 4.6, 1.6 Hz, 1H), 8.96 (dd, *J* = 8.1, 1.7 Hz, 1H), 8.54 (dd, *J* = 8.1, 1.7 Hz, 0.6H), 7.91 (dd, *J* = 8.1, 4.5 Hz, 1H), 7.81 (dd, *J* = 8.1, 4.5 Hz, 0.6H), 2.85 (s, two singlets are merged, 5H).

Mixture of isomers were separated by column chromatography.
first eluent isomer: ¹H NMR (300 MHz, CDCl₃) δ 9.17 (dd, *J* = 4.6, 1.7 Hz, 1H), 8.97 (dd, *J* = 8.1, 1.7 Hz, 1H), 7.91 (dd, *J* = 8.1, 4.6 Hz, 1H), 2.85 (s, 3H). ¹³C NMR (126 MHz, CDCl₃) δ 153.37, 151.67, 148.36, 142.07, 137.79, 131.81, 128.70, 121.23, 9.89.
second eluent isomer: ¹H NMR (300 MHz, CDCl₃) δ 9.25 (dd, *J* = 4.6, 1.6 Hz, 1H), 8.54 (dd, *J* = 8.3, 1.6 Hz, 1H), 7.81 (dd, *J* = 8.3, 4.6 Hz, 1H), 2.85 (s, 3H).

### Example 24

### 3-Methylpyrido[3,2-d][1,2,4]triazolo[4,3-b]pyridazine (minor isomer) 24a and 3-methylpyrido[2,3-d][1,2,4]triazolo [4,3-b]pyridazine 24b (major isomer)

The compound was prepared from compound **23** according to the example **20.** ¹H-NMR (500 MHz, CDCl₃): δ= 9.17 (dd, *J=* 4.7, 1.7 Hz, 1H), 9.06 (dd, *J* = 4.6, 1.7 Hz, 0.6H), 8.89 (dd, *J=* 8.2, 1.7 Hz, 0.6H), 8.83 (s, 0.6H), 8.67 (s, 1H), 8.28 (dd, *J* = 8.1, 1.7 Hz, 1H), 7.82 (dd, *J=* 8.1, 4.6 Hz, 0.6H), 7.73 (dd, *J=* 8.0, 4.6 Hz, 1H), 2.83 (broad signal, 4H). ¹³C-NMR (126 MHz, CDCl₃); δ= 155.78, 153.15, 148.68, 148.66, 148.38, 146.52, 142.80, 141.88, 141.07, 140.04, 135.59, 130.88, 127.69, 125.31, 120.41, 118.86, 9.89, 9.72. HRMS (ESI): cal. mass C₉H₈N₅ [M+1]⁺ 186.0774, found [M+1]⁺ 186.0772, cal. mass C₉H₇N₅ Na [M+Na]⁺ 208.0594, found [M+Na]⁺ 208.0594.

### Example 25

### Preparation for Single Isomer:

### 3-methylpyrido[3,2-d][1,2,4]triazolo[4,3-b]pyridazine 25

### furo[3,4-b]pyridin-5(7H)-one (vi)

2,3-pyridinedicarboxylic acid (2.0 g, 11.97 mmol, 1.0 equiv.) was dissolved in acetic acid anhydride (4.88 g, 47.87 mmol, 4 equiv.), and the mixture was heated to reflux and stirred for 4 h. Acetic anhydride was evaporated to dryness of quinolinic acid anhydride (1.82 g, 97%). The compound was subsequently used without further purification.

NaBH₄ (0.46 g, 12.15 mmol, 1.0 equiv.) was added to a solution of quinolinic acid anhydride (1.81 g, 12.15 mmol, 1.0 equiv.) in THF (10 mL) at 15 °C under argon. Acetic acid (1.45 g, 1.38 mL, 24.30 mmol, 2.0 equiv.) was added dropwise, and the resulting mixture was stirred at 15 °C for 4 h. The solvent was removed in vacuo. The residue was dissolved in acetic acid (6 mL) and acetic anhydride (6 mL), and the resulting solution was stirred for 3 h at 100 °C. The mixture was concentrated in vacuo and the residue was dissolved in NaCl solution (20 mL). The water phase was extracted with CHCl₃ (3 × 40 mL), and the combined organic layers were concentrated. Recrystallization from ^{*i*-}PrOH yielded compound (vi) (0.52 g, 32%) as a light-yellow solid. ¹H NMR (300 MHz, CDCl₃) δ 8.88 (dd, *J* = 4.9, 1.6 Hz, 1H), 8.22 (dd, *J* = 7.8, 1.6 Hz, 1H), 7.49 (dd, *J* = 7.8, 4.9 Hz, 1H), 5.35 (s, 2H). ¹³C NMR (75 MHz, CDCl₃) δ 169.11, 166.54, 155.39, 134.15, 124.00, 119.81, 70.61.

### 7-bromofuro[3,4-b]pyridin-5(7H)-one (vii)

Compound (vi) (0.500 g, 3.67 mmol, 1.0 equiv.) was heated at reflux with NBS (0.72 g, 4.04 mmol, 1.1 equiv.) and AIBN (10 mg) in dry CCl₄ (20 mL) for 2 h. The reaction mixture was cooled to room temperature, the succinate salts were filtered off, and the filtrate was concentrated and purified by silica gel column chromatography (DCM) to afford product (vii) (0.42 g 54%) as an oil. ¹H NMR (300 MHz, CDCl₃) δ 8.99 (dd, *J* = 4.9, 1.5 Hz, 1H), 8.25 (dd, *J* = 7.8, 1.6 Hz, 1H), 7.58 (dd, *J* = 7.8, 4.8 Hz, 1H), 7.39 (s, 1H).

### pyrido[2,3-d]pyridazin-5(6H)-one (viii)

Compound (vii) (0.38 g, 1.78 mmol, 1.0 equiv.) was dissolved in 5% HCl (5 mL/mmol) and stirred at reflux for 2 h, after which time the reaction was cooled to room temperature. Once cooled, the appropriate hydrazine (68.5 mg, 2.14 mmol, 1.2 equiv.) was added to the solution and the reaction stirred at room temperature for a further 2 h. The resulting precipitate was then collected and recrystallised from hot ethanol to afford the pure compound (viii) (0.17 g 66%). ¹H NMR (300 MHz, DMSO) δ 12.97 (s, 1H), 9.11 (dt, *J* = 4.6, 1.3 Hz, 1H), 8.57 (dd, *J* = 8.1, 1.7 Hz, 1H), 8.40 (s, 1H), 7.83 (ddd, *J* = 8.1, 4.6, 0.9 Hz, 1H).

### 5-chloropyrido[2,3-d]pyridazine (ix)

A mixture of compound (viii) (0.147 g, 1.0 mmol, 1.0 equiv.) and phosphorus oxychloride (0.382 g, 2.5 mmol, 2.5 equiv.) in pyridine (0.2 mL) was heated to reflux and stirred for 8 h. The mixture then was poured into a slush of 50 g of ice and then neutralized with NaHCO₃ and then extracted with ethyl acetate (3 × 100 mL). Organic phase was separated, washed with water, dried with Na₂SO₄ and evaporated to dryness. The crude product was purified by column chromatography using dichloromethane gave yellow amorphous solid (ix) (36 mg, 22%). ¹H NMR (300 MHz, CDCl₃) δ 9.73 (s, 1H), 9.32 (dd, *J=* 4.4, 1.6 Hz, 1H), 8.61 (dd, *J* = 8.5, 1.5 Hz, 1H), 7.93 (dd, *J* = 8.4, 4.4 Hz, 1H).

### 3-methylpyrido[3,2-d][1,2,4]triazolo[4,3-b]pyridazine 25

Compound (ix) (30 mg, 0.18 mmol, 1.0 equiv.) and acetic acid hydrazide (27 mg, 0.36 mmol, 2.0 equiv.) were refluxed in dioxane (3 mL) for 2 h. The reaction mixture was extracted with dichloromethane (4x20 mL). Organic phase was dried with Na₂SO₄ and evaporated. The crude product was purified by column chromatography using dichloromethane give amorphous solid (**25**) (21 mg, 65%). ¹H NMR (400 MHz, CDCl₃) δ 9.07 (dd, *J* = 4.6, 1.7 Hz, 1H), 8.91 (ddd, *J* = 8.2, 1.6, 0.7 Hz, 1H), 8.85 (d, *J* = 0.7 Hz, 1H), 7.82 (dd, *J* = 8.2, 4.6 Hz, 1H), 2.84 (s, 3H). ¹³C NMR (101 MHz, CDCl₃) δ 153.31, 148.86, 148.60, 142.08, 140.26, 131.10, 127.86, 120.65, 9.92.

### Example 26

### N,3-dimethylpyrido[3,4-d][1,2,4]triazolo[4,3-b]pyridazin-6-amine 26b (major isomer) and N,3-dimethylpyrido[4,3-d][1,2,4]triazolo[4,3-b]pyridazin-6-amine 26a (minor isomer)

To a solution of compound **19** (0.050 g, 0.22 mmol, 1.0 equiv.) in isopropanol (25 mL), then appropriate methylamine solution (30%) (0.5 mL) was added. The reaction mixture was refluxed for 3 h. Then, the mixture was cooled and poured onto ice water (20 mL). The formed precipitate was filtered, washed with water and crystallized from ethanol to afford mixture of isomers of **26a/26b** (ratio 0.7:1.0) (29 mg, 60%). ¹H NMR (600 MHz, DMSO) δ 9.61 (d, *J* = 0.9 Hz, 1H), 9.49 (d, *J* = 0.9 Hz, 0.7H), 8.97 - 8.96 (m, 1.7H), 8.20 (dd, *J* = 5.3, 0.9 Hz, 0.7H), 8.12 (dd, *J* = 5.7, 0.9 Hz, 1H), 7.98 - 7,85 (m, 1.7H), 2.96 (t, *J* = 4.6 Hz, 5H), 2.57 (d, *J* = 5.1 Hz, 5H). ¹³C NMR (126 MHz, DMSO) δ 151.85, 151.20, 150.73, 150.07, 147.05, 146.93, 146.51, 145.42, 139.60, 139.04, 128.82, 123.85, 117.99, 116.74, 115.32, 113.37, 28.19, 28.12, 9.32, 9.30. HRMS (ESI): cal. mass C₁₀H₁₀N₆ [M+1]⁺ 215.1040, found [M+1]⁺ 215.1041, cal. mass C₁₀H₁₀N₆Na [M+Na]⁺ 237.0859, found [M+Na]⁺ 237.0859.

### Example 27

### N-methytpyrido[2,3-d]tetrazolo[1,5-b]pyridazin-6-amine 27

To a solution of compound **21b** (0.050 g, 0.22 mmol, 1.0 equiv.) in isopropanol (25 mL), then appropriate methylamine solution (30%) (0.5 mL) was added. The reaction mixture was refluxed for 3 h. Then, the mixture was cooled and poured onto ice water (20 mL). The formed precipitate was filtered, washed with water and crystallized from ethanol to afford single isomer **27** (25 mg, 61%). ¹H NMR (600 MHz, DMSO) δ 9.15 (dd, *J* = 4.6, 1.6 Hz, 1H), 8.88 (dd, *J=* 8.1, 1.6 Hz, 1H), 8.44 (q, *J* = 4.9 Hz, 1H), 8.06 (dd, *J* = 8.1, 4.6 Hz, 1H), 3.03 (d, *J* = 4.9 Hz, 3H). ¹³C NMR (126 MHz, DMSO) δ 153.62, 153.18, 139.49, 136.44, 132.60, 128.23, 117.82, 28.04. HRMS (ESI): cal. mass C_{B}H₇N₇ [M+1]⁺ 202.0836, found [M+1]⁺ 202.0837, cal. mass C_{B}H₇N₇Na [M+Na]⁺ 224.0655, found [M+Na]⁺ 224.0655.

### Example 28

### 6-Ethoxy-3-methylpyrido[4,3-d][1,2,4]triazoio[4,3-b]pyridazine 28a (major isomer) and 6-ethoxy-3-methylpyrido [3,4-d][1,2,4]triazolo[4,3-b]pyridazine 28b (minor isomer)

Compound (**19**) (0.050 g, 0.22 mmol, 1.0 equiv.) was dissolved in ethanol (3 mL), then KCN (0.016 mg, 0.25 mmol, 1.1 equiv.) was added. The reaction mixture was refluxed for 4 h. Then, the mixture was cooled and poured onto ice water (20 mL) and extracted with DCM (3 × 20 mL). The organic solvent was evaporated under vacuum and crystallized from DCM to afford the product (**28a/28b**) (36 mg, 69%). ¹H NMR (300 MHz, CDCl₃) δ 9.90 (s, 1H), 9.45 (s, 0.5H), 9.03 (d, *J* = 5.4 Hz, 0.5H), 8.98 (d, *J* = 5.4 Hz, 1H), 8.34 (d, *J* = 5.4 Hz, 0.5H), 7.94 (d, *J* = 5.4 Hz, 1H), 4.61 (m, 3H), 2.74 (m, 5H), 1.58 (m, 5H). HRMS (ESI): cal. mass C₁₁H₁₁N₅O [M+1]⁺ 230.1036, found [M+1]⁺ 230.1037, cal. mass C₁₁H₁₁N₅ONa [M+Na]⁺ 252.0856, found [M+Na]⁺ 252.0852.

### Example 29

### 6-Azidopyrido[4,3-d]tetrazolo[1,5-b]pyridazine 29a and 6-azidopyrido[3,4-d]tetrazolo[1,5-b] pyridazine 29b

Compound (ii) (0.200 g, 1.0 mmol, 1.01 equiv.) was dissolved in acetonitrile (5 mL), then sodium azide (0.195 g, 3.02 mmol, 3.0 equiv.) was added. The reaction mixture was stirred for 24 h at room temperature. The precipitate was collected by filtration, washed with water, and dried in air yielded **29a/29b** (0.179g, 84%). ¹H NMR (600 MHz, CDCl₃): δ= 10.07 (d, *J* = 1.0 Hz, 1H), 9.60 (d, *J* = 1.0 Hz, 1H), 9.27 (d, *J* = 5.3 Hz, 1H), 9.22 (d, *J* = 5.4 Hz, 1H), 8.51 (dd, *J* = 5.3, 1.0 Hz, 1H), 8.03 (dd, *J* = 5.4, 1.0 Hz, 1H). HRMS (ESI): cal. mass C₇H₄N₉ [M+1]⁺ 214.0584, found [M+1]⁺ 214.0578, cal. mass C₉H₇N₅ Na [M+Na]⁺ 236.0404, found [M+Na]⁺ 236.0400.

### Example 30

### 3-Methyl-7,8,9,10-tetrahydropyrido[3,2-d][1,2,4]triazolo[4,3-b]pyridazine 30

A solution of compound **23** (50 mg) in methanol was pumped through a CatCart (Pd-C) using an ThalesNano H-Cube apparatus at a liquid flow rate of 1.0 mL/min at 60 °C. The crude product was purified by column chromatography using dichloromethane and methanol to give 32 mg (74%) of compound **30** as white amorphous solid. ¹H NMR (600 MHz, MeOD) δ 8.01 (s, 1H), 3.39 (ddd, *J* = 7.0, 4.0, 1.0 Hz, 2H), 2.85 (tt, *J* = 6.5, 1.0 Hz, 2H), 2.63 (s, 3H), 2.02 - 1.95 (m, 2H). ¹³C NMR (126 MHz, MeOD) δ 147.31, 146.78, 142.26, 139.08, 104.02, 41.37, 20.82, 20.41, 9.25. HRMS (ESI): cal. mass C₉H₁₂N₅ [M+1]⁺ 190.1087, found [M+1]⁺ 190.1092, cal. mass C₉H₁₁N₅ Na [M+Na]⁺ 212.0907, found [M+Na]⁺ 212.0908.

### Example 31

### 7,8,9,10-Tetrahydropyrido[3,2-d]tetrazolo[1,5-b]pyridazine 31a and 7,8,9,10-tetrahydropyrido[2,3-d]tetrazolo[1,5-b]pyridazine 31b

Prepared from 50 mg of compound **21** under the same conditions for the synthesis of example **30**, yield 30 mg (72%). ¹H-NMR (600 MHz, DMSO) δ 8.25 (s, 1H), 8.22 (s, 0.2H), 7.35 (s, 1H), 3.36 - 3.30 (m, 2H), 2.85 (t, *J* = 6.3 Hz, 2H), 2.73 (t, *J* = 6.2 Hz, 0.4H), 1.88 (h, *J* = 5.7 Hz, 2.4H).

### Example 32

### 3-methyl-7,8,9,10-tetrahydropyrido[4,3-d][1,2,4]triazolo[4,3-b]pyridazine 32a and 3-methyl-7,8,9,10-tetrahydropyrido[3,4-d][1,2,4]triazolo[4,3-b]pyridazine 32b

Prepared from 50 mg of compound **19** under the same conditions for the synthesis of example **30**, yield 25 mg (60%). ¹H NMR (300 MHz, CD₃OD_SPE) δ 8.36 (s, 1H), 8.34 (s, 0.5H), 4.25 (t, *J* = 2.1 Hz, 2H), 4.05 (t, *J* = 2.1 Hz, 1H), 3.29 - 3.21 (m, 3H), 3.14 - 3.03 (m, 1H), 2.88 - 2.83 (m, 2H), 2.76 (s, broad signal, 4H).

### Example 33

### 1-(9,10-dihydropyrido[4,3-d]tetrazolo[1,5-b]pyridazin-8(7H)-yl)ethan-1-one 33a and 1-(7,10-dihydropyrido[3,4-d]tetrazolo[1,5-b]pyridazin-9(8H)-yl)ethan-1-one 33b

### 7,8,9,10-tetrahydropyrido[4,3-d]tetrazolo[1,5-b]pyridazine (x)

Prepared from 50 mg of compound **17** under the same conditions for the synthesis of example **30**, yield compound (x) (22 mg, 52%).¹H NMR (300 MHz, DMSO) δ 8.77 (s, 0.5H), 8.76 (s, 1H), 4.03 (d, *J* = 2.4 Hz, 2H), 3.90 (s, 0H), 3.14 - 2.96 (m, 4H).

Compound (x) (1.0 equiv.) was dissolved in dichloromethane and added Et₃N (1.2 equiv.) and acetyl chloride (1.2 equiv.), stirred at room temperature for 6 h. The reaction mixture was extracted with DCM and water. The crude product was purified by column chromatography using DCM and MeOH (9:1) give amorphous solid **33a/33b** (25 mg, 48%). ¹H NMR (300 MHz, CDCl₃) δ 8.47 (s, 0.5H), 8.44 (s, 1H), 5.19 - 5.03 (m, 2H), 4.95 - 4.78 (m, 1H), 4.01 - 3.98 (m, 1H), 3.93 - 3.82 (m, 2H), 3.38 - 3.23 (m, 1H), 3.07 - 3.00 (m, 2H), 2.25 (s, broad signal, 5H).

### Example 34

### 3-Methylthieno[3,2-d][1,2,4]triazolo[4,3-b]pyridazine 34

### 2-formylthiophene-3-carboxylic acid

*n*-BuLi (15.60 mL, 39.01 mmol, 2.5 mol/L, 2.5 equiv.) was added dropwise to a stirring solution of commercially available thiophene-3-carboxylic acid **1** (2.0 g, 15.60 mmol, 1.0 equiv.) in dry THF (40 mL) at -78 °C. After 2 h, DMF (6 mL, 78.03 mmol, 5.0 equiv.) was added. After another 1 hour, the cooling bath was removed, and the mixture stirred at ambient temperature for 16 h. HCl (aq., 1 M, 60 mL) was added. The mixture was extracted with ethyl acetate (3 × 30 mL), dried (Na₂SO₄) and concentrated in vacuo. The resulting yellow solid (xi) (yield 2.20 g, 91%) was used without further purification. ¹H NMR (300 MHz, CDCl₃) δ 10.60 (s, 1H), 7.72 - 7.65 (m, 2H). HRMS (ESI): cal. mass C₆H₄O₃S [M+1]⁺ 156.9954, found [M+1]⁺ 156.9951, cal. mass C₆H₄O₃SNa [M+Na]⁺ 178.9773, found [M+Na]⁺ 178.9772.

### Thieno[2,3-d]pyridazin-4(5H)-one (xii)

A round-bottomed flask was equipped with a stir bar and reflux condenser. To the flask was added the product of (xi) (1.9 g, 12.16 mmol, 1.0 equiv.), hydrazine hydrate (55%) (1.52 g, 30.41 mmol, 2.5 equiv.), and EtOH (5 mL) and refluxed for 3 h. The reaction was cooled to room temperature and concentrated by rotary evaporation. Water (20 mL) was added and the filtrate was separated from the insoluble solids. The aqueous layer was concentrated by rotary evaporation to give a pale-yellow solid. The solid was dried in a vacuum oven overnight at 50 °C. Desired compound (xii) was obtained (1.56 g, 84% yield). ¹H NMR (500 MHz, DMSO) δ 12.84 (s, 1H), 8.57 (s, 1H), 8.04 (d, *J* = 5.2 Hz, 1H), 7.63 (d, *J* = 5.1 Hz, 1H). ¹³C NMR (126 MHz, DMSO) δ 158.38, 140.71, 135.85, 133.50, 133.08, 123.93. HRMS (ESI): cal. mass C₆H₄N₂OS [M+1]⁺ 153.0117, found [M+1]⁺ 153.0119, cal. mass C₆H₄N₂OSNa [M+Na]⁺ 174.9937, found [M+Na]⁺ 174.9936.

### 4-chlorothieno[2,3-d]pyridazine (xiii)

A round-bottomed flask was equipped with a stir bar and reflux condenser. To the flask was added the product of (xii) (1.3 g, 8.54 mmol, 1.0 equiv.), phosphorus oxychloride (8 mL, 85.43 mmol, 10.0 equiv.), and pyridine (1.4 mL, 17.08 mmol, 2.0 equiv.) and refluxed for 24 h. The reaction was cooled to r.t. and poured over ice and then neutralized with NaHCO₃. The mixture was separated and the aqueous layer was extracted with chloroform (4 × 30 mL). The organic layers were combined, dried (Na₂SO₄) and concentrated by rotary evaporation to give a yellow solid (xiii) (0.73 g, 50% yield). ¹H NMR (400 MHz, CDCl₃) δ 9.58 (d, *J* = 0.8 Hz, 1H), 7.96 (d, *J* = 5.3 Hz, 1H), 7.63 (dd, *J* = 5.3, 0.8 Hz, 1H). ¹³C NMR (101 MHz, CDCl₃) δ 151.33, 145.69, 140.39, 136.00, 134.23, 122.44. HRMS (EI): cal. mass C₆H₃ClN₂S [M]⁺ 169.9700, found [M]⁺ 169.9698.

### 3-methylthieno[3,2-d][1,2,4]triazolo[4,3-b]pyridazine 34

Compound (xiii) (75 mg, 0.43 mmol, 1.0 equiv.) and acetic acid hydrazide (65 mg, 0.87 mmol, 2.0 equiv.) were refluxed in dioxane (3 mL) for 3 h. The reaction mixture was extracted with dichloromethane (4 × 20 mL). Organic phase was dried with Na₂SO₄ and evaporated. The crude product was purified by column chromatography using dichloromethane give amorphous solid **34** (72 mg, 87%). ¹H NMR (400 MHz, CDCl₃) δ 8.73 (d, *J* = 0.7 Hz, 1H), 8.07 (dd, *J* = 5.2, 0.8 Hz, 1H), 8.00 (d, *J* = 5.3 Hz, 1H), 2.84 (s, 3H). ¹³C NMR (101 MHz, CDCl₃) δ 147.65, 142.51, 140.03, 134.99, 131.53, 130.12, 122.73, 10.28. HRMS (ESI): cal. mass C₈H₆N₄S [M+1]⁺ 191.0386, found [M+1]⁺ 191.0388, cal. mass C₈H₆N₄SNa [M+Na]⁺ 213.0205, found [M+Na]⁺ 213.0208.

### Example 35

### Tetrazolo[1,5-b]thieno[3,2-d]pyridazine 35

Compound (xiii) (70 mg, 0.41 mmol, 1.0 equiv.) was dissolved in DMF (1 mL), then sodium azide (32 mg, 0.48 mmol, 1.2 equiv.) was added. The reaction mixture heated at 120°C for 3 h. The reaction was cooled to r.t. and poured into water. The mixture was separated and the aqueous layer was extracted with chloroform (4 × 20 mL). The organic layers were combined, dried (Na₂SO₄) and concentrated by rotary evaporation to give a yellow amorphous solid **35** (70 mg, 97% yield). ¹H NMR (300 MHz, DMSO) δ 9.55 (s, 1H), 8.63 (d, *J* = 5.2 Hz, 1H), 8.19 (d, *J* = 5.2 Hz, 1H). ¹³C NMR (75 MHz, DMSO) δ 143.62, 141.48, 140.39, 134.60, 130.26, 122.42. HRMS (ESI): cal. mass C₆H₃N₅S [M+1]⁺ 178.0182, found [M+1]⁺ 178.0184, cal. mass C₆H₃N₅SNa [M+Na]⁺ 200.0001, found [M+Na]⁺ 200.0006.

### Example 36

### 3-methylthieno[2,3-d][1,2,4]triazolo[4,3-b]pyridazine 36

### 3-formylthiophene-2-carboxylic acid (xiv)

The compound (xiv) was prepared from thiophene-2-carboxylic acid according to the compound (xi) in example **34.** ¹H NMR (300 MHz, CDCl₃) δ 10.48 (s, 1H), 7.64 - 7.59 (m, 2H). HRMS (ESI): cal. mass C₆H₄O₃S [M+1]⁺ 156.9954, found [M+1]⁺ 156.9948, cal. mass C₆H₄O₃SNa [M+Na]⁺ 178.9773, found [M+Na]⁺ 178.9770.

### Thieno[2,3-d]pyridazin-7(6H)-one (xv)

The compound (xv) was prepared from compound (xiv) according to the compound (xii) in example **34.** ¹H NMR (500 MHz, DMSO) δ 12.93 (s, 1H), 8.44 (s, 1H), 8.19 (d, *J* = 5.1 Hz, 1H), 7.58 (d, *J* = 5.1 Hz, 1H). ¹³C NMR (126 MHz, DMSO) δ 158.19, 139.97, 136.24, 135.83, 134.55, 124.82. HRMS (ESI): cal. mass C₆H₄N₂OS [M+1]⁺ 153.0117, found [M+1]⁺ 153.0118, cal. mass C₆H₄N₂OSNa [M+Na]⁺ 174.9937, found [M+Na]⁺ 174.9942.

### 7-chlorothieno[2,3-d]pyridazine (xvi)

The compound (xvi) was prepared from compound (xv) according to the compound (xiii) in example **34.** ¹H NMR (600 MHz, CDCl₃) δ 9.49 (s, 1H), 7.94 (d, *J* = 5.3 Hz, 1H), 7.58 (d, *J* = 5.3 Hz, 1H). ¹³C NMR (126 MHz, CDCl₃) δ 150.89, 146.07, 139.51, 138.28, 134.53, 123.26. HRMS (ESI): cal. mass C₆H₃ClN₂S [M+1]⁺ 170.9778, found [M+1]⁺ 170.9781, cal. mass C₆H₃ClN₂SNa [M+Na]⁺ 192.9598, found [M+Na]⁺ 192.9596.

### 3-methylthieno[2,3-d][1,2,4]triazolo[4,3-b]pyridazine 36

The compound **36** was prepared from compound (xvi) according to the compound **34.** ¹H NMR (600 MHz, CDCl₃) δ 8.69 (s, 1H), 7.73 (d, J= 5.2 Hz, 1H), 7.55 (d, *J* = 5.2 Hz, 1H), 2.85 (s, 3H). ¹³C NMR (126 MHz, CDCl₃) δ 147.76, 142.17, 141.23, 132.51, 130.76, 129.98, 124.00, 10.31. HRMS (ESI): cal. mass C₈H₆N₄S [M+1]⁺ 191.0386, found [M+1]⁺ 191.0388, cal. mass C₈H₆N₄SNa [M+Na]⁺ 213.0205, found [M+Na]⁺ 213.0207.

### Example 37

### Tetrazolo[1,5-b]thieno[2,3-d]pyridazine 37

The compound **37** was prepared from compound (xvi) according to the compound **35.** ¹H NMR (500 MHz, DMSO) δ 9.43 (s, 1H), 8.43 (d, *J* = 5.1 Hz, 1H), 7.99 (d, J= 5.1 Hz, 1H). ¹³C NMR (126 MHz, DMSO) δ 144.02, 141.33, 134.94, 134.78, 130.73, 125.22. HRMS (ESI): cal. mass C₆H₃N₅S [M+1]⁺ 178.0182, found [M+1]⁺ 178.0183, cal. mass C₆H₃N₅SNa [M+Na]⁺ 200.0001, found [M+Na]⁺ 200.0003.

### Example 38

### 6-chloro-3-methylthieno[3,4-d][1,2,4]triazolo[4,3-b]pyridazine 38

### Dimethyl thiophene-3,4-dicarboxylate (xvii)

A round-bottomed flask was equipped with a stir bar and reflux condenser. The flask was charged with the commercially available diacid (2.5 g, 14.52 mmol, 1.0 equiv.) and dissolved in MeOH (65 mL) with a catalytic amount of H₂SO₄ (~2.5 mL). The reaction was heated to reflux and stirred for 24 h. The reaction was cooled to r.t. and concentrated rotary evaporation. The residue was purified by silica gel chromatography column and was eluted DCM to yield dimethyl thiophene-3,4-dicarboxylate (xvii) as a solid (2.42 g, 83%). ¹H NMR (400 MHz, CDCl₃) δ 7.85 (s, 2H), 3.88 (s, 6H). ¹³C NMR (101 MHz, CDCl₃) δ 163.62, 133.37, 131.97, 52.49. HRMS (ESI): cal. mass C₈H₈O₄S [M+1]⁺ 201.0216, found [M+1]⁺ 201.0216, cal. mass C₈H₈O₄SNa [M+Na]⁺ 223.0036, found [M+Na]⁺ 223.0040.

### 2,3-dihydrothieno[3,4-d]pyridazine-1,4-dione (xviii)

Compound (xvii) (2.0 g, 0.9.99 mmol, 1.0 equiv.) was charged in a round bottomed flask in ethanol (15 mL) and allowed to react with hydrazine hydrate (1.35 g, 26.97 mmol, 2,7 equiv.). The reaction mixture was refluxed for 4 h. After the reaction was completed, the reaction mixture was cooled to room temperature and concentrated under vacuum. The crude solid compound was dissolved in a minimum amount of water containing 1% NH₄OH by volume and precipitated by adding concentrated HCl to the solution. Finally, the obtained solid compound was washed with cold water, MeOH and then dried at room temperature to give (xviii) as a solid (1.3 g, 78%). ¹H NMR (400 MHz, DMSO) δ 11.20 (broad singlet, 2H), 8.36 (s, 2H). ¹³C NMR (101 MHz, DMSO) δ 153.12, 130.42, 128.41. HRMS (ESI): cal. mass C₆H₄N₂O₂S [M+1]⁺ 169.0066, found [M+1]⁺ 169.0069, cal. mass C₆H₄N₂O₂SNa [M+Na]⁺ 190.9886, found [M+Na]⁺ 190.9891.

### 1,4-dichlorothieno[3,4-d]pyridazine (xix)

A round-bottomed flask was equipped with a stir bar and reflux condenser. To the flask was added the product (xviii) (1.3 g, 7.73 mmol, 1.0 equiv.), phosphorus oxychloride (4 mL), and pyridine (1mL) and refluxed for 16 h under Argon. The reaction was cooled to r.t. and poured over ice. The mixture was separated and the aqueous layer was extracted with chloroform (4 x 75 mL). The organic layers were combined, dried (Na₂SO₄) and concentrated by rotary evaporation. The residue was purified by silica gel chromatography column and was eluted DCM to yield (xix) as a yellow solid (0.72 g, 46%). ¹H NMR (400 MHz, CDCl₃) δ 8.31 (s, 2H). ¹³C NMR (126 MHz, CDCl₃) δ 150.37, 131.24, 126.40. HRMS (ESI): cal. mass C₆H₂N₂SCl₂ [M+1]⁺ 204.9389, found [M+1]⁺ 204.9389, cal. mass C₆H₂N₂SCl₂Na [M+Na]⁺ 226.9208, found [M+Na]⁺ 226.9209.

### 6-chloro-3-methylthieno[3,4-d][1,2,4]triazolo[4,3-b]pyridazine 38

Compound (xix) (0.204 g, 1.0 mmol, 1.0 equiv.) and acetohydrazine (0.222 g, 3.0 mmol, 2.0 equiv.) were refluxed in dioxane (3 mL) for 16 h. The reaction mixture was extracted with Chloroform (4 × 30 mL). Organic phase was dried with Na₂SO₄ and evaporated. The crude product was purified by column chromatography using dichloromethane give amorphous solid **38** (0.192 g, 86%). ¹H NMR (600 MHz, CDCl₃) δ 8.42 (d, J= 3.0 Hz, 2H), 8.28 (d, *J* = 3.1 Hz, 2H), 2.75 (s, 6H). ¹³C NMR (126 MHz, CDCl₃) δ 147.78, 145.11, 141.19, 129.60, 127.87, 124.31, 122.63, 10.20. HRMS (ESI): cal. mass C₈H₅N₄SCl [M+1]⁺ 224.9996, found [M+1]⁺ 224.9997, cal. mass C₈H₅N₄SClNa [M+Na]⁺ 246.9816, found [M+Na]⁺ 246.9822.

### Example 39

### 3-methylthieno[3,4-d][1,2,4]triazolo[4,3-b]pyridazine 39

Compound **38** (40 mg, 0.17 mmol, 1.0 equiv.) was dissolved ethanol (3 mL) and the hydrazine hydride (55%) (17.8 mg, 0.35 mmol, 2.0 equiv.) was added slowly and the reaction mixture was refluxed for 2 h to give precipitation. The product was collected by suction filtration and dried. Then, Solution of 0.15 M TMSOK (22 mg, 0.17 mmol, 1.0 equiv.) in water (1.6 mL) was added, slurry was stirred for 48 h at room temperature. The resulting mixture was extracted with Chloroform (4 × 20 mL). Organic phase was dried with Na₂SO₄ and evaporated to give amorphous solid **39** (12 mg, 35%). ¹H NMR (600 MHz, CDCl₃) δ 8.49 (d, *J* = 0.9 Hz, 1H), 8.39 (dd, *J* = 2.9, 0.9 Hz, 1H), 8.15 (d, *J* = 2.9 Hz, 1H), 2.76 (s, 3H). ¹³C NMR (126 MHz, CDCl₃) δ 148.18, 142.27, 141.73, 128.44, 128.31, 124.58, 121.39, 10.26. HRMS (ESI): cal. mass C₈H₆N₄S [M+1]⁺ 191.0386, found [M+1]⁺ 191.0387, cal. mass C₈H₆N₄SNa [M+Na]⁺ 213.0205, found [M+Na]⁺ 213.0199.

### Example 40

### 6-chlorotetrazolo[1,5-b]thieno[3,4-d]pyridazine 40

1,4-dichlorothieno[3,4-d]pyridazine (xix) (0.100 g, 0.49 mmol, 1.0 equiv.) was dissolved methanol (8 mL) and the hydrazine hydride (55%) (0.049 g, 0.98 mmol, 2.0 equiv.) was added slowly and the reaction mixture was refluxed for 1 h. After the reaction was completed, the reaction mixture was cooled to room temperature and concentrated under vacuum to give crude yellow precipitation. Which was dissolved in acetic acid (5 mL, 2N) and under stirring the ice-cold solution was treated with a cold aqueous solution of NaNO₂ (50 mg, 0.73 mmol, 1.5 equiv. in 2 mL water) dropwise. The reaction mixture was stirred at room temperature for 1 h. The compound was extracted with chloroform (3 × 25 mL). Organic phase was dried with Na₂SO₄and evaporated to give amorphous solid **40** (48 mg, 46%). ¹H NMR (500 MHz, DMSO) δ 8.99 (d, J= 2.8 Hz, 1H), 8.80 (d, J= 2.8 Hz, 1H). ¹³C NMR (126 MHz, DMSO) δ 150.35, 140.43, 130.79, 126.37, 124.04, 121.94.

### Example 41

### Tetrazolo[1,5-b]thieno[3,4-d]pyridazine 41

Compound **40** (40 mg, 0.18 mmol, 1.0 equiv.) was dissolved ethanol (3 mL) and the hydrazine hydride (55%) (18.9 mg, 0.37 mmol, 2.0 equiv.) was added slowly and the reaction mixture was refluxed for 2 h to give precipitation. The product was collected by suction filtration and dried. Then, Solution of 0.15 M TMSOK (24 mg, 0.18 mmol, 1.0 equiv.) in water (1.7 mL) was added, slurry was stirred for 48 h at room temperature. The resulting mixture was extracted with Chloroform (4 × 20 mL). Organic phase was dried with Na₂SO₄ and evaporated to give amorphous solid **41** (8 mg, 23%). ¹H NMR (600 MHz, CDCl₃) δ 9.34 (s, 1H), 8.48 (d, *J* = 3.2 Hz, 1H), 7.96 (d, *J* = 3.2 Hz, 1H). ¹³C NMR (126 MHz, CDCl₃) δ 157.22, 135.32, 132.41, 131.03, 130.35, 126.09. HRMS (ESI): cal. mass C₆H₃N₅S [M+1]⁺ 178.0182, found [M+1]⁺ 178.0192.

### Example 42

### 3-methylfuro[2,3-d][1,2,4]triazolo[4,3-b]pyridazine 42

### 2-formylfuran-3-carboxylic acid (xx)

*n*-BuLi (17.84 mL, 44.61 mmol, 2.5 mol/L, 2.5 equiv.) was added dropwise to a stirring solution of commercially available furan-2-carboxylic acid (2.0 g, 17.84 mmol, 1.0 equiv.) in dry THF (60 mL) at -78 °C. After 2 h, DMF (2.7 mL, 35.68 mmol, 2.0 equiv.) was added. After another 1 hour, the cooling bath was removed, and the mixture stirred at ambient temperature for 16 h. Then, HCl (aq., 1 M, 60 mL) was added. The mixture was extracted with ethyl acetate (3 × 40 mL), dried (Na₂SO₄) and concentrated in vacuo. The resulting yellow solid (xx) (yield 2.36 g, 94%) was used without further purification.

### 7-chlorofuro[2,3-d]pyridazine (xxi)

A round-bottomed flask was equipped with a stir bar and reflux condenser. To the flask was added the product of 2-formylfuran-3-carboxylic acid (xx) (0.500 g, 3.57 mmol, 1.0 equiv.), hydrazine hydrate (55%) (0.446 g, 8.92 mmol, 2.5 equiv.), and MeOH (10 mL) and refluxed for 4 h. The reaction was cooled to room temperature and concentrated by rotary evaporation and dried under high vacuum to give curde furo[2,3-d]pyridazin-7(6H)-one. Then, phosphorus oxychloride (5.46 g, 3.3 mL, 35.71 mmol, 10.0 equiv.), and pyridine (0.56 g, 0.57 mL, 7.14 mmol, 2.0 equiv.) and refluxed for 24 h. The reaction was cooled to r.t. and poured over ice and then neutralized with NaHCO₃. The mixture was separated and the aqueous layer was extracted with chloroform (4 × 50 mL). The organic layers were combined, dried (Na₂SO₄) and concentrated by rotary evaporation. The residue was purified by silica gel chromatography column and was eluted DCM to yield a yellow solid 7-chlorofuro[2,3-d]pyridazine (xxi) (0.135 g, 25% yield). ¹H NMR (400 MHz, CDCl₃) δ 9.47 (s, 1H), 7.94 (d, J= 2.1 Hz, 1H), 7.00 (d, J= 2.1 Hz, 1H). ¹³C NMR (126 MHz, CDCl₃) δ 149.84, 149.15, 146.51, 141.89, 105.74. HRMS (EI): cal. mass C₆H₃ClN₂O [M]⁺ 153.9928, found [M]⁺ 153.9927.

### 3-methylfuro[2,3-d][1,2,4]triazolo[4,3-b]pyridazine 42

7-chlorofuro[2,3-d]pyridazine (xxi) (25 mg, 0.16 mmol, 1.0 equiv.) and acetohydrazine (24 mg, 0.32 mmol, 2.0 equiv.) were refluxed in dioxane (2 mL) for 24 h. The reaction mixture was extracted with chloroform (4 × 20 mL). Organic phase was dried with Na₂SO₄ and evaporated. The crude product was purified by column chromatography using dichloromethane give 3-methylfuro[2,3-d][1,2,4]triazolo [4,3-b]pyridazine **42** as an amorphous solid (17 mg, 60%). ¹H NMR (400 MHz, CDCl₃) δ 8.66 (s, 1H), 7.90 (d, J= 2.1 Hz, 1H), 7.00 (d, J= 2.1 Hz, 1H), 2.85 (s, 3H). ¹³C NMR (126 MHz, CDCl₃) δ 147.96, 147.31, 145.54, 140.93, 138.11, 117.60, 106.72, 10.39. HRMS (ESI): cal. mass C₈H₆N₄O [M+1]⁺ 175.0614, found [M+1]⁺ 175.0617, cal. mass C₈H₆N₄ONa [M+Na]⁺ 197.0434, found [M+Na]⁺ 197.0434.

### Example 43

### furo[2,3-d]tetrazolo[1,5-b]pyridazine 43

7-chlorofuro[2,3-d]pyridazine (xxi) (50 mg, 0.32 mmol, 1.0 equiv.) was dissolved in DMF (1 mL), then sodium azide (31 mg, 0.48 mmol, 1.5 equiv.) was added. The reaction mixture heated at 120 °C for 24 h. The reaction was cooled to r.t. and poured into water. The mixture was separated and the aqueous layer was extracted with chloroform (4 × 25 mL). The organic layers were combined, dried (Na₂SO₄) and concentrated by rotary evaporation to give a furo[2,3-d]tetrazolo[1,5-b]pyridazine **43** as a yellow amorphous solid (40 mg, 76% yield). ¹H NMR (400 MHz, CDCl₃) δ 9.01 (s, 1H), 8.14 (d, J= 2.1 Hz, 1H), 7.23 (d, J= 2.1 Hz, 1H). ¹³C NMR (101 MHz, CDCl₃) δ 149.91, 144.73, 142.13, 137.04, 122.11, 107.26. HRMS (ESI): cal. mass C₆H₃N₅O [M+1]⁺ 162.0410, found [M+1]⁺ 162.0409, cal. mass C₆H₃N₅ONa [M+Na]⁺ 184.0230, found [M+Na]⁺ 184.0235.

### Example 44

### 6,10-dichloro-3-methyl-[1,2,4]triazolo[3,4-a]phthalazine 44a (major isomer) and 6,7-dichloro-3-methyl-[1,2,4]triazolo[3,4-a]phthalazine 44b (minor isomer)

### 5-chloro-2,3-dihydrophthalazine-1,4-dione

4-chloroisobenzofuran-1,3-dione (10 g, 54.76 mmol, 1.0 equiv.) was dissolved in 10% HCl (50 mL), and hydrazine hydrate (55%) (2.63g, 82.14 mmol, 1.5 equiv.) was added and the mixture was refluxed with stirring for 16 h. The product was collected by suction filtration, washed with water and dried, to yield an amorphous solid (10.1 g, 93%). ¹H NMR (300 MHz, DMSO) δ 11.60 (s, 2H), 8.00 (d, *J* = 7.6 Hz, 1H), 7.84 (m, 2H). HRMS (ESI): cal. mass C₈H₅ClN₂O₂ [M+1]⁺ 197.0112, found [M+1]⁺ 197.0110, cal. mass C₈H₅ClN₂O₂ [M+Na]⁺ 218.9932, found [M+Na]⁺ 218.9938.

### 1,4,5-trichlorophthalazine

A round-bottomed flask was equipped with a stir bar and reflux condenser. To the flask was added the 5-chloro-2,3-dihydrophthalazine-1,4-dione (10 g, 50.86 mmol, 1.0 equiv.), phosphorus oxychloride (23.65 mL, 508.64 mmol, 5.0 equiv.) and refluxed for 24 h. The reaction was cooled to r.t. and poured over ice. The mixture was separated and the aqueous layer was extracted with chloroform (4 × 60 mL). The organic layers were combined, dried (Na₂SO₄) and concentrated by rotary evaporation. The crude product was purified by column chromatography using dichloromethane give amorphous solid (2.1 g, 26%). ¹H NMR (400 MHz, CDCl₃) δ 8.34 (dd, *J* = 8.3, 1.2 Hz, 1H), 8.11 (dd, *J* = 7.8, 1.2 Hz, 1H), 7.94 (dd, *J* = 8.3, 7.8 Hz, 1H). ¹³C NMR (126 MHz, CDCl₃) δ 155.28, 152.45, 137.79, 134.17, 132.06, 129.45, 125.83, 124.59. HRMS (ESI): cal. mass C₈H₃Cl₃N₂ [M+1]⁺ 232.9435, found [M+1]⁺ 232.9441, cal. mass C₈H₃Cl₃N₂ [M+Na]⁺ 254.9254, found [M+Na]⁺ 254.9263.

### 6,10-dichloro-3-methyl-[1,2,4]triazolo[3,4-a]phthalazine 44a (major isomer) and 6,7-dichloro-3-methyl-[1,2,4]triazolo[3,4-a]phthalazine 44b (minor isomer)

1,4,5-trichlorophthalazine (500 mg, 2.15 mmol, 1.0 equiv.) and acetic acid hydrazide (318 mg, 4.31 mmol, 2.0 equiv.) were refluxed in dioxane (6 mL) for 4 h. The reaction mixture was extracted with dichloromethane (4 × 40 mL). Organic phase was dried with Na₂SO₄ and concentrated by rotary evaporation to give a solid (421 mg, 77%). ¹H NMR (300 MHz, CDCl₃) δ 8.67 (dd, *J* = 7.4, 2.0 Hz, 0.17H), 8.23 (dd, *J* = 8.1, 1.1 Hz, 1H), 8.03 (dd, *J* = 8.0, 1.2 Hz, 1H), 7.88 - 7.80 (m, 0.36H), 7.77 (t, *J* = 8.1 Hz, 1H), 2.83 (s, 3H), 2.81 (s, 0.60H). HRMS (ESI): cal. Mass C₁₀H₆Cl₂N₄ [M+1]⁺ 253.0042, found [M+1]⁺ 253.0042, cal. Mass C₁₀H₆Cl₂N₄ [M+Na]⁺ 274.9862, found [M+Na]⁺ 274.9868.

Mixture of isomers were separated by column chromatography. First eluent isomer: 6,10-dichloro-3-methyl-[1,2,4]triazolo[3,4-a]phthalazine **44a** (major isomer) ¹H NMR (300 MHz, CDCl₃) δ 8.23 (dd, *J* = 8.1, 1.3 Hz, 1H), 8.04 (dd, *J* = 8.1, 1.3 Hz, 1H), 7.77 (t, *J* = 8.1Hz, 1H), 2.83 (s, 3H). second eluent isomer: 6,7-dichloro-3-methyl-[1,2,4]triazolo[3,4-a]phthalazine **44b** (minor isomer) ¹H NMR (300 MHz, CDCl₃) δ 8.68 (dd, *J* = 7.3, 1.9 Hz, 1H), 7.91 - 7.80 (m, 2H), 2.81 (s, 3H).

### Example 45

### 10-chloro-3-methyl-[1,2,4-]triazolo[3,4-a]phthalazin 45

Compound **44a** (major isomer) (250 mg, 0.98 mmol, 1.0 equiv.) was dissolved ethanol (5 mL) and the hydrazine hydride (55%) (63.24 mg, 1.97 mmol, 2.0 equiv.) was added slowly and the reaction mixture was refluxed for 3 h to give precipitation. The product was collected by suction filtration and dried. Then, Solution of 0.15 M TMSOK (126 mg, 0.98 mmol, 1.0 equiv.) in water was added, slurry was stirred for 48 h at room temperature. The resulting mixture was extracted with Chloroform (4 × 30 mL). Organic phase was dried with Na₂SO₄ and evaporated. The crude product was purified by column chromatography using dichloromethane give amorphous solid (45 mg, 21%). ¹H NMR (300 MHz, CDCl₃) δ 8.59 (s, 1H), 7.98 (dd, *J* = 7.9, 1.2 Hz, 1H), 7.84 (dd, *J* = 7.8, 1.2 Hz, 1H), 7.71 (t, *J* = 7.9 Hz, 1H), 2.84 (s, 3H). HRMS (ESI): cal. mass C₁₀H₇ClN₄ [M+1]⁺ 219.0432, found [M+1]⁺ 219.0435, cal. mass C₁₀H₇ClN₄ [M+Na]⁺ 241.0251, found [M+Na]⁺ 241.0259.

### Example 46

### 6,10-dichlorotetrazolo[5,1-a]phthalazine 46

1,4,5-trichlorophthalazine (800 mg, 3.44 mmol, 1.0 equiv.) was dissolved ethanol (10 mL) and the hydrazine hydride (55%) (221 mg, 6.89 mmol, 2.0 equiv.) was added slowly and the reaction mixture was refluxed for 1 h. After the reaction was completed, the reaction mixture was cooled to room temperature and concentrated under vacuum to give crude yellow precipitation. Which was dissolved in acetic acid (15 mL, 2N) and under stirring the ice-cold solution was treated with a cold aqueous solution of NaNO₂ (356 mg, 5.17 mmol, 1.5 equiv. in 2 mL water) dropwise. The reaction mixture was stirred at room temperature for 1 h. The compound was extracted with chloroform (3 × 35 mL). Organic phase was dried with Na₂SO₄ and evaporated to give an amorphous solid (452 mg, 55%). ¹H NMR (300 MHz, CDCl₃) δ 8.43 (dd, *J* = 8.3, 1.1 Hz, 1H), 8.21 (dd, *J* = 8.0, 1.1 Hz, 1H), 8.01 (t, *J* = 8.1 Hz, 1H). HRMS (ESI): cal. mass C₈H₃Cl₂N₅ [M+1]⁺ 239.9838, found [M+1]⁺ 239.9842, cal. mass C₈H₃Cl₂N₅ [M+Na]⁺ 261.9658, found [M+Na]⁺ 261.9664.

### Example 47

### 10-chlorotetrazolo[5,1-a]phthalazine 47

This was prepared from compound **46** according to the procedure given in example **45.** ¹H NMR (300 MHz, CDCl₃) δ 8.95 (s, 1H), 8.16 (dd, J = 7.9, 1.2 Hz, 1H), 8.07 (dd, J = 8.0, 1.2 Hz, 1H), 7.95 (t, J = 7.9 Hz, 1H).

### Biological results

### Example 59

### Cell culture

**Compound reconstitution.** Prior to treatment, each compound was used from a stock solution of a concentration of 10 mg/ml in DMSO. Final concentration of DMSO in cell cultures was kept below ...

**Cell culture and treatment.** The human renal fibroblast cell line TK173 was derived from a normal human kidney (laboratory stock; (G. A. Müller et al., Exp. Nephrol. 1995, 3, 127). Cell line stabilization for research purposes was approved by the Ethics Committee of the Georg-August-University. TK173 cells were cultured in Dulbecco's modified Eagle's medium (DMEM, Gibco, USA) supplemented with 10% fetal bovine serum (FBS, Gibco, Germany) and 1% penicillin-streptomycin (PS, Gibco, USA) at 37°C in a humidified atmosphere with 5% CO₂. Cell line was routinely tested for mycoplasma contamination. To investigate the effect of compounds after profibrotic agent treatment, 3×10⁴ TK173 cells were seeded in one well of a 6-well culture plate in normal medium. After 24 hours, the culture medium was removed, and the cells were incubated for a further 24 hours in FBS free culture medium (starvation medium). Then, 10 ng/ml TGFβ1 (R&D Systems, USA) was added to the starvation medium, and the culture medium was changed with fresh TGFβ1 in every alternate day. After 5 days of TGFβ1 treatment, cell culture medium was replaced by starvation medium with compounds at different concentration for following 48 hours of incubation.

### Example 60

### Enzyme-linked immunosorbent assay (ELISA) to detect secreted Type I Collagen.

Conditioned media containing the secretome of TK173 fibroblast were harvested after 48 hours of compound treatments. Secretion of pro-Collagen 1 was assessed with Human Pro-Collagen I alpha 1 DuoSet ELISA (R&D Systems, USA). To prepare the ELISA plate, Human Pro-Collagen I α1 Capture Antibody was diluted in ELISA Plate-coating Buffer at 4 µg/ml working concentration. 100 µl of diluted Capture Antibody was added to the 96-well microplates and incubated overnight at room temperature. After three times washing with Wash Buffer, microplates were blocked by adding 300 µl of Reagent Diluent for 1 hour at room temperature. To proceed the ELISA, Human Pro-Collagen I α1 Standards were diluted in Reagent Diluent (range between 31,3 pg/ml - 2000 pg/ml) and 100 µl of standards or conditioned mediums were add into designated wells. Microplates were incubated at room temperature for 2 hours. Human Pro-Collagen I α1 Detection Antibody was prepared in Reagent Diluent at 100 ng/ml working concentration and 100 µl of diluted Detection Antibody was applied for 2 hours at room temperature after extensive washing. Then, working solution of Streptavidin-HRP with Reagent Diluent was prepared by diluting 1:40 and added into each well. After 20 minutes at room temperature incubation, microplates were washed and applied with 100 µl of Substrate Solution for 20 minutes followed by Stop Solution after washing. Finally, signals were measured at 450 nm absorbance by PHOmo Microplate Reader (Autobio, China). The amount of pro-collagen 1 antibody fusion was quantified by extrapolating the signal into a linear range of a pro-collagen 1 standard curve. Screening compounds were typically tested in at concentrations between 5 and 200 µg/ml. The IC₅₀ values observed by the dose response curves for each compound (n = 4-6, two independent experiments) were obtained by nonlinear regression analysis using Prism 9 software (GraphPad, La Jolla, USA) and represent the concentration where the TGFβ-stimulated collagen secretion is attenuated by 50%.

Selected compound data (µg/ml):
**1:** 157; **2:** 58; **3:** 96; **5:** 39; **6:** 61; **7:** 32; **9:** 10; **10:** 93; **13:** 87; **15:** 105; **23:** 21; **35:** 30; **38:** <80; **39:** <80; **41:** <60.

### Example 61

### Collagen I mRNA PCR assay

**RNA isolation.** Total RNA was extracted by the TRIzol reagent (Life technologies, USA) and purified by PureLink RNA Mini Kit (Ambion, USA) according to instructions available from the manufacturer with minor modifications. The samples were stored at -80°C prior to use. RNA concentrations were measured by NanoDrop Spectrophotometer (ThermoFisher Scientific, USA), and were equilibrated to 100 ng for complementary DNA (cDNA) synthesis.

**Quantitative real-time PCR analysis (qPCR).** For SYBR Green-based qPCR analysis, cDNA preparation was performed by using DNAse I (Sigma-Aldrich, USA) digestion to remove residual genomic DNA and SuperScript II Reverse Transcriptase kit (Invitrogen, USA) to synthesize cDNA following the manufacturer's detailed instructions. All qPCR reactions were performed using the StepOne Plus Real-Time System (Applied Biosystems, USA) and amplifications were done using the SYBR Green PCR Master Mix (Applied Biosystems, USA). The thermal cycling conditions were initiated to 95°C for 20 seconds, 40 amplification cycles at 95°C for 3 seconds and 60°C for 30 seconds, with one cycle of dissociation at 95°C for 15 seconds, 60°C for 60 seconds, and 95°C for 15 seconds. Three replicates per sample were run in duplicate. The experimental cycle threshold (Ct) values were normalized to those obtained for GAPDH, and the relative gene expression level was determined using the 2_ddCT methods. Primer sequences were listed in the Table 1.

**Table 1.**

| **Primer** | **Sequence** |
|---|---|
| **human GAPDH-F** | **5' - ACCCACTCCTCCACCTTTGAC - 3'** |
| **human GAPDH-R** | **5' - TCCACCACCCTGTTGCTGTAG - 3'** |
| **human ACTA2-F** | **5' - ACTGAGCGTGGCTATTCCTCCGTT - 3'** |
| **human ACTA2-R** | **5' - GCAGTGGCCATCTCATTTTCA - 3'** |
| **human Collagen 1-F** | **5' - GTACTGGATTGACCCCAACC - 3'** |
| **human Collagen 1-R** | **5' - CGCCATACTCGAACTGGAAT - 3'** |

Selected compound data (IC₅₀, µg/ml):
**1:** <80; **3:** <20; **5:** <20; **12:** <50;

### Example 62

### Collagen I Western Blot

**Protein Extraction.** Total proteins were extracted from the cultured cells by NP40 Cell Lysis Buffer (Invitrogen, USA) containing phosphatase (Sigma-Aldrich, USA) and protease inhibitor (Roche, Switzerland) followed by sonication and centrifugation at 12,000 x g for 20 minutes at 4°C to clear the lysates. The supernatant was collected and measured using Pierce BCA Protein Assay Kit (ThermoFisher Scientific) by PHOmo Microplate Reader (Autobio, China). The samples were stored at -80°C prior to use.

**Immunoblotting.** Western blot analysis, 10 µg of extracted proteins were diluted in NuPAGE LDS Samples Buffer (Novex Life technologies, USA) containing 5% 2-Mercaptoethanol (Sigma-Aldrich, Germany), and following denatured for 5 minutes at 95°C. The proteins were resolved by 4-12% NuPAGE SDS-PAGE (Novex Life technologies, USA). Blots were often stripped with Thermo Scientific Restore PLUS Western Blot Stripping Buffer (Thermo Fisher Scientific, Germany) for reprobing. The following antibodies were used for immunoblotting: Collagen I (1:1000; ab233080, Abcam, USA), alpha smooth muscle Actin (1:1000; ab124964, Abcam, USA), GAPDH (1:8000; sc-32233, Santa Cruz, USA). Proteins were detected by LumiGLO chemiluminescent system (Cell Signaling Technology, USA) with appropriate secondary antibodies: anti-mouse HRP (1:2500; Dako, Denmark) and anti-rabbit HRP (1:2500; 7074, Cell Signaling Technology, USA) using ChemiDoc MP Imaging System (Bio-Rad, USA).

Selected compound data (IC₅₀, µg/ml):
**1:** <40; **3:** <5; **6:** <5; **9:** <1; **12:** <5; **14:** <30; **15:** <40; **27:** < 30; **35:** < 40

### Cited literature:

Document 1: Leask, Andrew. "Potential therapeutic targets for cardiac fibrosis: TGFβ, angiotensin, endothelin, CCN2, and PDGF, partners in fibroblast activation." Circulation research 106.11 (2010): 1675-1680.
Document 2: Mozaffarian, Dariush, et al. "Heart disease and stroke statistics-2016 update: a report from the American Heart Association." circulation 133.4 (2016): e38-e360.
Document 3: Hinderer, Svenja, and Katja Schenke-Layland. "Cardiac fibrosis-A short review of causes and therapeutic strategies." Advanced drug delivery reviews 146 (2019): 77-82.
Document 4: Fan, Zhaobo, and Jianjun Guan. "Antifibrotic therapies to control cardiac fibrosis." Biomaterials research 20.1 (2016): 1-13.
Document 5: Frangogiannis, Nikolaos G. "Cardiac fibrosis: cell biological mechanisms, molecular pathways and therapeutic opportunities." Molecular aspects of medicine 65 (2019): 70-99.
Document 6: Gourdie, Robert G., Stefanie Dimmeler, and Peter Kohl. "Novel therapeutic strategies targeting fibroblasts and fibrosis in heart disease." Nature reviews Drug discovery 15.9 (2016): 620-638.
Document 7: Shibasaki, Yasunobu, et al. "Impact of the angiotensin II receptor antagonist, losartan, on myocardial fibrosis in patients with end-stage renal disease: assessment by ultrasonic integrated backscatter and biochemical markers." Hypertension research 28.10 (2005): 787-795.
Document 8: B Pitt, Bertram, et al. "Cardiovascular events with finerenone in kidney disease and type 2 diabetes." New England Journal of Medicine 385.24 (2021): 2252-2263.
Document 9: Spinale, Francis G. "Myocardial matrix remodeling and the matrix metalloproteinases: influence on cardiac form and function." Physiological reviews 87.4 (2007): 1285-1342.
Document 10: Oyamada, Shizu, et al. "Chymase inhibition reduces infarction and matrix metalloproteinase-9 activation and attenuates inflammation and fibrosis after acute myocardial ischemia/reperfusion." Journal of Pharmacology and Experimental Therapeutics 339.1 (2011): 143-151.
Document 11: Hayashidani, Shunji, et al. "Anti-monocyte chemoattractant protein-1 gene therapy attenuates left ventricular remodeling and failure after experimental myocardial infarction." Circulation 108.17 (2003): 2134-2140.
Document 12: Bujak, Marcin, et al. "Induction of the CXC chemokine interferon-γ-inducible protein 10 regulates the reparative response following myocardial infarction." Circulation research 105.10 (2009): 973-983.
Document 13: Rodriguez-Pascual, F., and O. Busnadiego. "Gonzalez-Santamaria J." Life Sci 118.2 (2014): 156.
Document 14: Daskalopoulos, Evangelos P., et al. "Targeting the Wnt/frizzled signaling pathway after myocardial infarction: a new tool in the therapeutic toolbox?." Trends in cardiovascular medicine 23.4 (2013): 121-127.
Document 15: Porter, Karen E., and Neil A. Turner. "Cardiac fibroblasts: at the heart of myocardial remodeling." Pharmacology & therapeutics 123.2 (2009): 255-278.
Document 16: Caulfield, J. B., and T. K. Borg. "The collagen network of the heart." Laboratory investigation; a journal of technical methods and pathology 40.3 (1979): 364-372.
Document 17: Hales, Patrick W., et al. "Histo-anatomical structure of the living isolated rat heart in two contraction states assessed by diffusion tensor MRI." Progress in biophysics and molecular biology 110.2-3 (2012): 319-330.
Document 18: Zeisberg, Elisabeth M., et al. "Endothelial-to-mesenchymal transition contributes to cardiac fibrosis." Nature medicine 13.8 (2007): 952-961.
Document 19: Tampe, Björn, et al. "Induction of Tet3-dependent epigenetic remodeling by low-dose hydralazine attenuates progression of chronic kidney disease." EBioMedicine 2.1 (2015): 19-36.
Document 20: Tampe, Björn, et a/. "Induction of Tet3-dependent epigenetic remodeling by low-dose hydralazine attenuates progression of chronic kidney disease. " EBioMedicine 2.1 (2015): 19-36*.*
Document 21: Batchelor, J. R., et al. "Hydralazine-induced systemic lupus erythematosus: influence of HLA-DR and sex on susceptibility." The lancet 315.8178 (1980): 1107-1109.
Document 22: Shepherd, Alexander MM, et al. "Hydralazine kinetics after single and repeated oral doses." Clinical Pharmacology & Therapeutics 28.6 (1980): 804-811.
Document 23: Bourdi, Mohammed, et al. "Anti-liver microsomes autoantibodies and dihydralazine-induced hepatitis: specificity of autoantibodies and inductive capacity of the drug." Molecular pharmacology 42.2 (1992): 280-285.
Document 24: Chang, Christopher, and M. Eric Gershwin. "Drugs and autoimmunity-a contemporary review and mechanistic approach." Journal of autoimmunity 34.3 (2010): J266-J275.
Document 25: Lodyga, Monika, and Boris Hinz. "TGF-β1-a truly transforming growth factor in fibrosis and immunity." Seminars in cell & developmental biology. Vol. 101. Academic Press, (2020): 123-139
Document 26: Carthy, Jonathon M. "TGFβ signaling and the control of myofibroblast differentiation: Implications for chronic inflammatory disorders." Journal of cellular physiology 233.1 (2018): 98-106.
Document 27: Lijnen, P. J., V. V. Petrov, and R. H. Fagard. "Induction of cardiac fibrosis by transforming growth factor-β1." Molecular genetics and metabolism 71.1-2 (2000): 418-435.
Document 28: Müller, G. A., et al. "Human renal fibroblast cell lines (tFKIF and tNKF) are new tools to investigate pathophysiologic mechanisms of renal interstitial fibrosis." Experimental nephrology 3.2 (1995): 127-133.
Document 29: Druey, J., and B. H. Ringier. "Hydrazinderivate der Phtalazin-und Pyridazinreihe." Helvetica Chimica Acta 34.1 (1951): 195-210.
Document 30: Zimmer, Hans, John M. Kokosa, and K. J. Shah. "Synthesis of condensed heterocyclic systems. VI. Ring closure reactions involving 1-hydrazinophthalazine." The Journal of Organic Chemistry 40.20 (1975): 2901-2906.
Document 31: Badr, M. Z. A., et al. "Substitution and ring closure reactions of phthalazine derivatives." Journal of heterocyclic chemistry 21.2 (1984): 471-475*.*
Document 32: Müller, G. A., et al. "Human renal fibroblast cell lines (tFKIF and tNKF) are new tools to investigate pathophysiologic mechanisms of renal interstitial fibrosis." Experimental nephrology 3.2 (1995): 127-133.

### Patent documents:

WO 2016/085981 A1
WO 2016/046130 A1
WO 2021/089828 A1
DE 102019126517 A1

## Claims

1. Azolo compound (I) 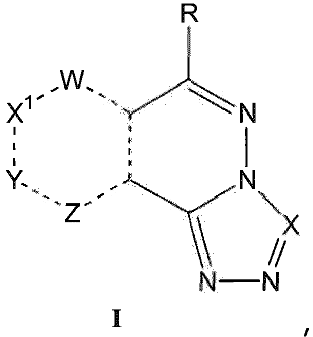 wherein
R is selected from the group consisting of H, D, CN, NHR⁵, NDR⁵, Cl, OR³, SR³ and C₁₋₄-alkyl, in particular H, D, methyl;
X is N or CR¹, wherein the nitrogen or carbon atom is an aromatic atom;
W is selected from the group consisting of a covalent bond, CR⁴, wherein the carbon atom is an aromatic ring atom, CH₂, S, O, N, wherein the nitrogen atom is an aromatic ring atom, NH and NR²;
X¹ is selected from the group consisting of a covalent bond, CR⁴, wherein the carbon atom is an aromatic ring atom, CH₂, S, O, N, wherein the nitrogen atom is an aromatic ring atom, NH and NR²;
Y is selected from the group consisting of a covalent bond, CR⁴, wherein the carbon atom is an aromatic ring atom, CH₂, S, O, N, wherein the nitrogen atom is an aromatic ring atom, NH and NR²;
Z is selected from the group consisting of a covalent bond, CR⁴, wherein the carbon atom is an aromatic ring atom, CH₂, S, O, N, wherein the nitrogen atom is an aromatic ring atom, NH and NR²;
wherein 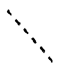 denotes a single bond or a double bond; wherein
W, X¹, Y and Z are members of the same ring and are selected such that the ring consists of five or six members;
W, X¹, Y and Z are selected such that the ring does not comprise more than two nitrogen atoms in the ring, and W, X¹, Y and Z are selected such that the ring does not comprise an acetal, thioacetal, aminal or hydrazine group;
wherein the compound does not comprise a peroxide or disulfide group; and wherein
R¹ is selected from the group consisting of H, D, CD₃, CHD₂, CH₂D, CF₃, CHF₂, CH₂F, CDF₂, CD₂F, C₁₋₄-alkyl, optionally substituted with OH, halogen, OR³;
R² is selected from the group consisting of CHO, COCH₃, COC₂H₅, COC₃H₇, COCH(CH₃)₂, COC₄H₉, COC(CH₃)₃;
R³ is selected from the group consisting of C₁₋₄-alkyl;
R⁴ is selected from the group consisting of H, D, F, Cl, methyl, CH₂F, CHF₂, CF₃, OCH₃, OCD₃; and
R⁵ is C₁₋₄-alkyl, optionally substituted with OH,
for use in a method of treatment or prevention of fibrosis.

2. The azolo compound (I) for use according to claim 1, wherein R is selected from the group consisting of H, CN, NHR⁵, Cl, OR³, methyl, ethyl, propyl, isopropyl, butyl, isobutyl, *sec*-butyl, *tert-butyl,* cyclopropyl and SR³.

3. The azolo compound (I) for use according to claim 1 or claim 2, wherein R¹ is selected from the group consisting of H, D, CD₃, CHD₂, CH₂D, CF₃, CHF₂, CH₂F, methyl, ethyl, propyl, isopropyl, butyl, isobutyl, *sec-butyl, tert-butyl* and cyclopropyl, optionally substituted by OH, Halogen or OR³, optionally, wherein R¹ is selected from the group consisting of H, methyl, CD₃, CF₃, CH₂OH, ethyl, CH₂OCH₃ and cyclopropyl,
and/or wherein R³ is selected from the group consisting of methyl, ethyl, propyl, isopropyl, butyl, isobutyl, *sec*-butyl and *tert-butyl,*
and/or wherein R⁵ is selected from the group consisting of methyl, ethyl, propyl, isopropyl, butyl, isobutyl, *sec-butyl, tert-butyl,* optionally substituted by OH.

4. The azolo compound (I) for use according to any of claims 1 to 3, wherein W, X¹, Y and Z are selected such that the compound does not include a peroxide group and/or such that the compound does not include a disulfide group.

5. The azolo compound (I) for use according to any of claims 1 to 4, wherein X is selected from the group consisting of N and CR¹ and wherein R¹ is selected from the group consisting of H, methyl, CH₂F, CD₂F, CHF₂, CDF₂, and CF₃, preferably, wherein X is CR¹ and R is H or D.

6. The azolo compound (I) for use according to any of claims 1 to 5, wherein W, X¹, Y and Z are selected such that the ring consists of carbon atoms or such that the ring consists of carbon atoms and one heteroatom,
and/or wherein W, X¹, Y and Z are selected such that the ring is aromatic or non-aromatic.

7. The azolo compound (I) for use according to any of claims 1 to 6 wherein W, X¹, Y and Z are selected to form a moiety according to one of the following ring atom sequence: C···C···C···C, N···C···C···C, C···N···C···C, C···C···N···C, C···C···C···N, S···C···C···C, C···S···C···C, C···C···S···C, C···C···C···S, O···C···C···C, C···O···C···C, C···C···O···C, C···C···C···O, C···C···C, N···C···C, C···N···C, C···C···N, S···C···C, C···S···C, C···C···S,, O···C···C, C···O···C, C···C···O, wherein "···" denotes a single bond or a double bond, and/or
wherein W, X¹, Y and Z are independently selected such that the ring forms a pyrrolidine, dihydrofuran, dihydrothiophen, pyrrole, furan, thiophene, oxazole, thiazole, tetrahydrobenzene, oxane, thiane, benzene, pyridine, pyran, thiopyran, preferably benzene, pyridine, dihydropyridine, thiophene or furan structure.

8. The azolo compound (I) for use according to any one of claims 1 to 7, wherein W, X¹, Y and Z are independently selected to form a moiety selected from the group consisting of CH=CH-CH=CH, CH=N-CH=CH, N=CH-CH=CH, CH=CH-CH=N, NH-CH₂-CH₂-CH₂, CH₂-NH-CH₂=CH₂, CH₂-NCOCH₃-CH₂-CH₂, S-CH=CH, CH=CH-S, CH-S-CH, CH=CH-O, preferably CH=CH-CH=CH or CH-S-CH.

9. The azolo compound (I) for use according to any one of claims 1 to 8,
wherein W, X¹, Y and Z are selected to form the moiety CH=CH-CH=CH and wherein R = H and wherein X is N; or
wherein W, X¹, Y and Z are selected to form the moiety CH=CH-CH=CH and wherein R = H and wherein X is C-CH₃; or
wherein W, X¹, Y and Z are selected to form the moiety CH=CH-CH=CH and wherein R = H and wherein X is C-H; or
wherein W, X¹, Y and Z are selected to form the moiety CH-S-CH and wherein R = H and wherein X is C-CH₃; or
wherein W, X¹, Y and Z are selected to form the moiety CH-S-CH and wherein R = H and wherein X is N.

10. The azolo compound (I) for use according to any one of claims 1 to 9, wherein X is C-CH₃, R is H, and W, X¹, Y and Z are forming the group CR⁴=CR⁴-CR⁴=CR⁴, wherein one R⁴ group is selected from the group consisting of F, Cl and OCH₃ and wherein the remaining three R⁴ groups are hydrogens.

11. The azolo compound (I) for use according to any one of claims 1 to 10, wherein X is C-CH₃, R is H, and W, X¹, Y and Z are forming the group CR⁴=CR⁴-CR⁴=CR⁴, wherein two R⁴ groups are independently selected from the group consisting of F, Cl and OCH₃ and wherein the remaining two R⁴ groups are hydrogens.

12. The azolo compound (I) for use according to any one of claims 1 to 11, wherein the azolo compound (I) is selected from the group consisting of: 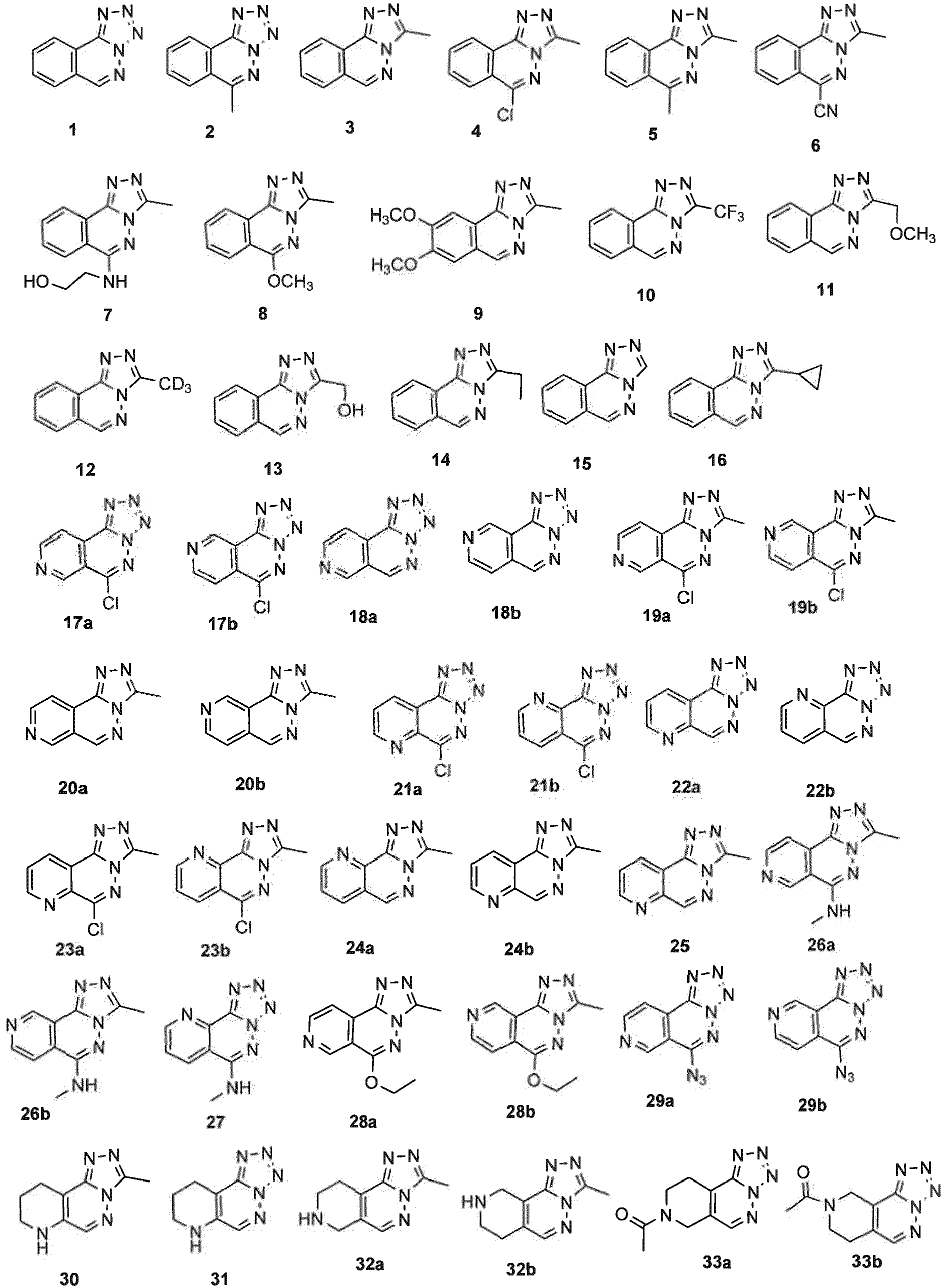 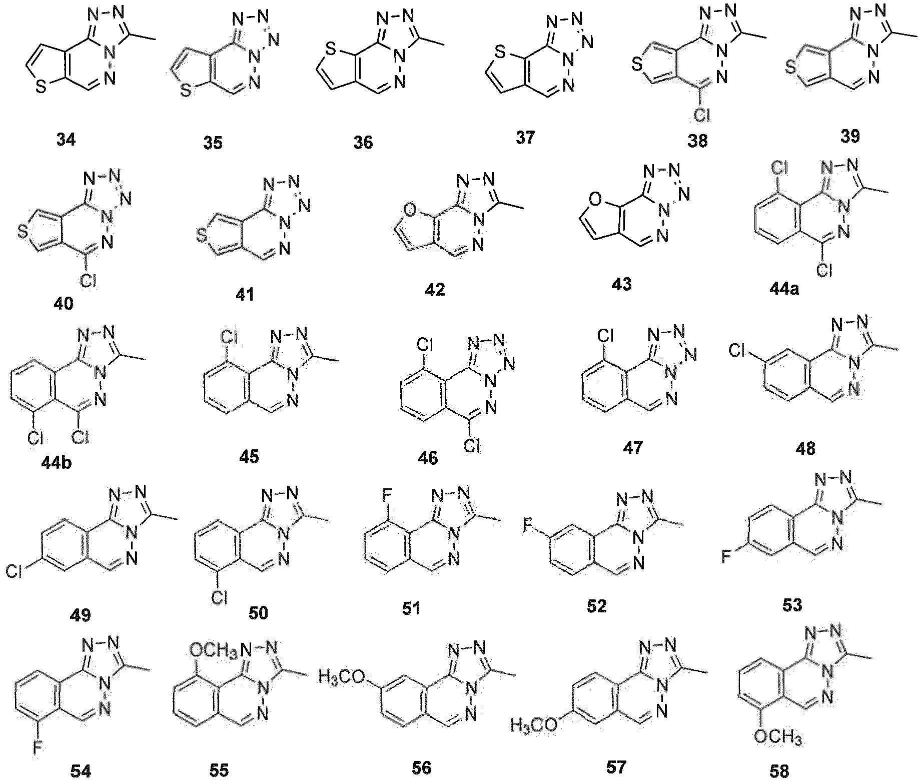 , optionally, wherein the azolo compound (I) is selected from the group consisting of:

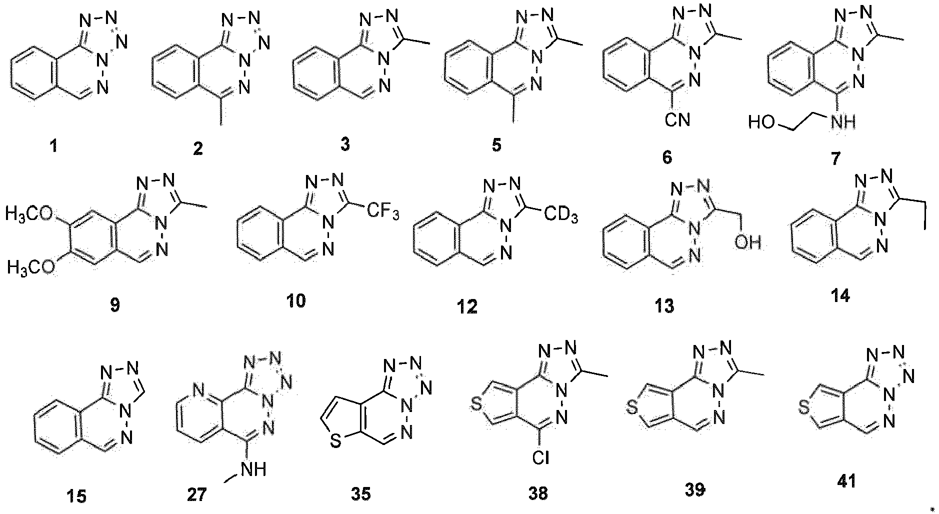

13. The azolo compound (I) for use according to any of claims 1 to 12, wherein fibrosis is selected from the group consisting of kidney fibrosis, liver fibrosis, lung fibrosis and cardiac fibrosis, optionally cardiac fibrosis.

14. Pharmaceutical composition comprising an azolo compound or a mixture of azolo compounds each having the structure (I) 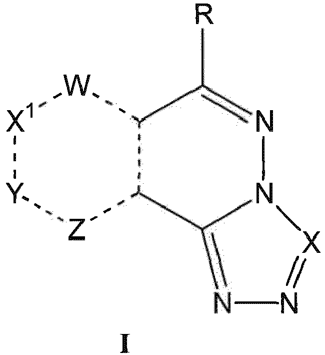 as defined in claim 1 and a pharmaceutically acceptable carrier for use in the treatment of fibrosis.

15. The pharmaceutical composition according to claim 14, which is an oral solid dosage form and/or wherein the azolo compound is present in an amount of from 5 to 500 mg, preferably 10 to 200 mg.
